# EUROPEAN PATENT SPECIFICATION

(11) **EP 3 512 591 B1**
(45) Date of publication and mention of the grant of the patent: **07.10.2026**
(21) Application number: 17777709.1
(22) Date of filing: 14.09.2017
(51) Int. Cl.: A61M 25/06, A61M 25/10

(54) **LUBRICIOUS INSERTION TOOLS FOR MEDICAL DEVICES**
SCHMIERENDE EINSETZWERKZEUGE FÜR MEDIZINISCHE VORRICHTUNGEN
OUTILS D'INSERTION LUBRIFIANTS POUR DISPOSITIFS MÉDICAUX

(30) Priority: 16.09.2016 US 201662395610 P; 28.02.2017 US 201762464520 P
(43) Date of publication of application: 24.07.2019
(73) Proprietor: Surmodics, Inc., Eden Prairie, MN 55344 (US)
(72) Inventor: TROCKE, Amy, Plymouth, Minnesota 55446 (US); SUTTON, Gregg S., Orono, Minnesota 55364 (US); SLAGER, Joram, St. Louis Park, Minnesota 55416 (US)
(74) Representative: Schiweck Weinzierl Koch Patentanwälte Partnerschaft mbB
(86) International application number: PCT/US2017/051555
(87) International publication number: WO 2018/053124

(56) References cited:
- WO-A1-2011/075727
- WO-A1-2012/161994
- WO-A1-2016/115361
- WO-A2-2014/179767
- US-A- 4 921 479
- US-A1- 2008 183 202
- US-A1- 2013 096 604
- US-A1- 2015 217 092

## Description

### Field

The disclosure relates to insertion tools that facilitate the insertion of a medical device into the vasculature of the body.

### Background

Atherosclerosis is a disease that affects arteries of the body, with most cases affecting the coronary arteries. When occurring in arteries in the leg, either above or below the knee, the disease is often referred to as peripheral arterial disease (PAD). During the onset of atherosclerosis, changes in the walls of the arteries are seen characterized by increases in cholesterol content and scar tissue. Later on, atherosclerotic plaques build up and thicken the wall of the artery, forming lesions that often cause arterial narrowing or stenosis resulting in reduced a blood flow. At these later stages, calcium can be present in the plaques.

As a general matter, it is desired to treat patients found to have plaques because, whether the plaque impedes blood flow or not, their presence presents a risk of rupture which could trigger a coronary event. A ruptured plaque can stimulate local formation of a blood clot that can block the flow of blood. In coronary arteries this will cause myocardial infarction. In peripheral vessels this can cause severe pain and, if occurring in multiple vessels, may lead to critical limb ischemia. See, e.g. WO2011075727 A1 and WO2014179767 A2.

### Summary of the Invention

The invention is defined by independent claim 1 and subsequent dependent claims 2 - 7.

### Summary of the Disclosure

Described herein are insertion tools for medical devices that are introduced into the vasculature, and which can be used to treat arterial diseases such as atherosclerotic plaques. The insertion tools can be used for packaging and protecting an implantable or insertable medical device during storage and deployment. The insertion tools can also be used to protect and facilitate the insertion of a medical device such as a balloon catheter. The insertion tool may facilitate balloon catheter insertion through a hemostatic valve and into a patient catheter lumen, and can protect the surface of a balloon member of the balloon during loading. The insertion tool can also protect the device from contamination, and/or minimize or prevent loss of coating from the balloon catheter during insertion into the body.

Insertion tools of the present disclosure can also be used to protect health care professionals from having contact with drug coatings on medical devices that are inserted into the human body. Furthermore, the insertion tools can also act to protect humidity sensitive drug coatings and prevent accidental contact with fluids with the drug coatings prior to insertion into the body of a mammal.

The insertion tool can be a part of a kit or system used for a medical procedure, which are also embodiments of the disclosure. For example, the kit can include one or more of the following components: one or more delivery catheters, a balloon treatment device, an inflation catheter, a guidewire, a hemostatic valve, or combinations thereof.

In one embodiment, the disclosure provides an insertion tool configured to facilitate entry of a balloon portion of a balloon catheter into a patient's body through a hemostatic valve. The insertion tool includes: proximal and distal ends along a lengthwise axis; a tubular portion extending proximally from the distal end having a length that is least the length of the balloon portion length, and a tab at the proximal end. The tubular portion includes a wall, an inner diameter that can accommodates a balloon portion of a balloon catheter, a separation margin in the wall of a first lengthwise half of the tubular portion and which represents a structural weakening of, or a split in the wall in the first half. The tab at the proximal end is either (a) a tab that extends from a second lengthwise half of the tubular portion, the tab having a portion at an angle skew to the lengthwise axis, or a tab (b) in the form of a solid article comprising a concave surface that is fastened to an outer surface of the second lengthwise half of the tubular portion.

The disclosure also provides a balloon catheter insertion system comprising: (i) a balloon catheter comprising a balloon portion having a length, (ii) a hemostatic valve, and (iii) an insertion tool that facilitates entry of the balloon portion of the balloon catheter into a patient's body through the hemostatic valve, as described herein.

The disclosure also provides a method, not claimed, for inserting a balloon catheter in a patient's body. The method includes steps of: (a) providing a (i) a balloon catheter comprising balloon portion having a length and a catheter shaft proximal to the balloon portion (ii) an insertion tool to facilitate entry of the balloon catheter into a patient's body, the insertion tool as described herein; (b) inserting the distal end of the insertion tool in a hemostatic valve; (c) advancing the tubular portion of the insertion tool and balloon catheter therein through the hemostatic valve and into the patient's body; (d) withdrawing the tubular portion of the insertion tool from the hemostatic valve so as to position at least a proximal portion of the tubular portion around the catheter shaft; and (e) moving the insertion tool in relation to the balloon catheter to cause the separation margin to separate so the insertion tool can be moved away from the catheter shaft.

In another embodiment, the disclosure provides an insertion tool configured to facilitate entry of a balloon portion of a balloon catheter into a patient's body through a hemostatic valve, where the insertion tool includes proximal and distal ends along a lengthwise axis; a tubular portion extending proximally from the distal end having a length that is at least the length of the balloon portion length, the tubular portion including a wall, an inner diameter that can accommodate a balloon portion of a balloon catheter, first and second separation margins in the wall of the tubular portion representing structural weakenings of, or splits in the wall of the tubular portion, wherein the first and second separation margins define first and second lengthwise halves of the tubular portion; and a first tab that extends from the first lengthwise half of the tubular portion and a second tab that extends from the second lengthwise half of the tubular portion. The disclosure also provides a balloon catheter insertion system that includes this insertion tool, and also a method, not claimed, for inserting a balloon catheter in a patient's body, the method using this insertion tool or the system that includes the tool.

### Brief Description of the Drawings

Figure 1 is an illustration of an insertion tool according to one embodiment of the disclosure.
Figure 2 is an illustration of a portion of an insertion tool, as viewed from the distal end, according to one embodiment of the disclosure.
Figure 3 is an illustration of a cross section of an insertion tool according to one embodiment of the disclosure.
Figure 4 is an illustration of a distal end of an insertion tool according to one embodiment of the disclosure.
Figure 5A is an illustration of a distal end of an insertion tool according to one embodiment of the disclosure.
Figure 5B is an illustration of a cross section of a portion of a distal end of an insertion tool according to one embodiment of the disclosure.
Figures 6A-6D are illustrations of proximal end embodiments of insertion tools.
Figure 7 is an illustration of a proximal or distal end embodiment of an insertion tool.
Figure 8 is an illustration of a proximal or distal end embodiment of an insertion tool.
Figure 9 is an illustration of a portion of an insertion tool, as viewed from the distal end, according to one embodiment of the disclosure.
Figure 10 is an illustration of a portion of an insertion tool, as viewed from the proximal end, according to one embodiment of the disclosure.
Figure 11 is an illustration of a portion of an insertion tool, as viewed from the proximal end, according to one embodiment of the disclosure.
Figure 12 is an illustration of a perspective view of an inventive insertion tool.
Figure 13 is an illustration of a distal end of an insertion tool according to one embodiment of the disclosure.
Figure 14 is an illustration of portions of a balloon catheter, according to one embodiment of the disclosure.
Figure 15 is an illustration of a distal end a balloon catheter showing a balloon portion in a folded configuration.
Figure 16 are illustration of a distal end of a balloon catheter showing a balloon portion in a folded configuration and the balloon portion loaded into various insertion tool embodiments.
Figure 17 is an illustration of a portion of a balloon catheter within an insertion tool.
Figure 18 is an illustration of a portion of a balloon catheter within an insertion tool, and a portion of the insertion tool within a hemostatic valve.

### Description

The embodiments of the present invention described herein are not intended to be exhaustive or to limit the invention to the precise forms disclosed in the following detailed description. Rather, the embodiments are chosen and described so that others skilled in the art can appreciate and understand the principles and practices of the present invention.

The publications and patents disclosed herein are provided solely for their disclosure.

The terms "proximal" and "distal" are used herein to define the location of certain features of the balloon catheter insertion tool, or associated system components such as a balloon catheter or a hemostatic valve. The proximal end ("user end") refers to location of a feature of the system that is towards the user, i.e., towards the outside the body. The distal end ("treatment end") refers to location of a feature of the system that is away from the user end, i.e., towards the treatment site. A "proximal portion" refers to a portion that is more towards the proximal end relative to a portion that is more towards the distal end, which is a "distal portion." The "inner surface" ("luminal surface") refers to the surface of an article that is within the lumen of a hollow article, whereas the "outer surface" refers to the surface on the outside of the hollow article ("abluminal surface"). Likewise, the inner surface of such an article can define an "inner diameter," and the outer surface can define an "outer diameter," wherein the difference between the outer and inner diameters can define a "wall thickness," such as the thickness of the wall of the insertion tool. The arrangement of features of the insertion tool can also be explained with regards to a "lengthwise axis" of the tool which is a line including points at the proximal and distal ends of the tool and running parallel with the wall of the insertion tool.

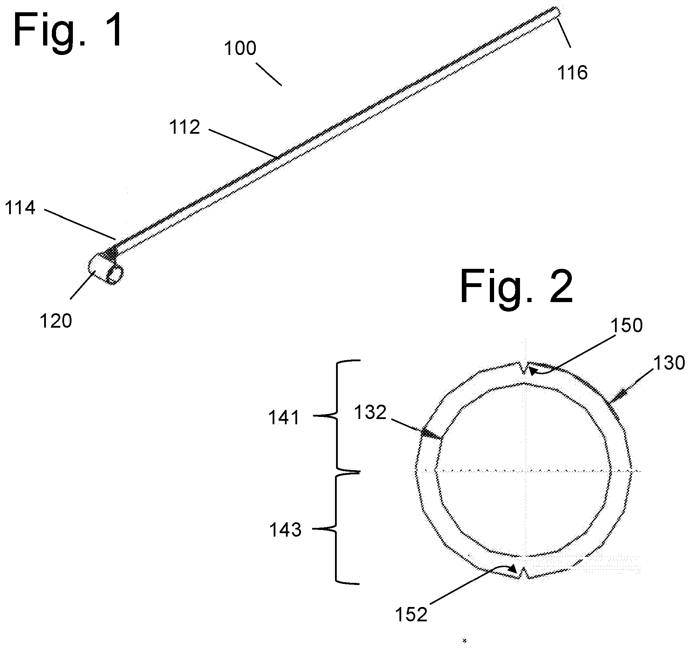

With reference to Figure 1, in one embodiment the disclosure provides a balloon catheter insertion tool **100** that includes a tubular portion **112** having proximal **114** and distal **116** ends. The tubular portion **112** has a lengthwise axis parallel with the wall of the tubular portion **112,** and portions of the insertion tool may be described with regard to the relationship to the lengthwise axis. The tubular portion can have a length, i.e., the distance between the proximal **114** and distal **116** ends, which can accommodate the length of the balloon portion of a balloon catheter. The insertion tool can be used to house and facilitate insertion of balloon catheters of various shapes and sizes. For example, the length of the tubular portion can be at least about 10 mm, such as in the range of about 10 mm to about 300 mm; other exemplary ranges are in the range of about 10 mm to about 100 mm, about 50 mm to about 150 mm, and about 150 mm to about 300 mm.

Figure 2 shows a view of the tubular portion of an insertion tool from its distal end **116.** Although the tubular portion is shown as having a circular shape as viewed from the end, it can be of any shape (e.g, oval or polygonal) suitable for accommodating a balloon portion of a balloon catheter, and facilitating entry through a hemostatic valve.

Figure 2 also shows the outer surface **130** and the inner surface of the **132** tubular portion. The distance between two points on the outer surface opposite each other, through the center of the tubular portion, represents the outer diameter (OD). The distance between two points on the inner surface opposite each other, through the center of the tubular portion, represents the inner diameter (ID). In exemplary embodiments the outer diameter of the tubular portion can be in the range of about 0.35 mm to about 10 mm, about 1.5 mm to about 5 mm, or about 1.6 mm to about 3 mm. In exemplary embodiments the circumference of the tubular portion can be in the range of about 1.1 mm to about 32 mm, about 2.5 mm to about 15 mm, or about 3 mm to 6 mm. In exemplary embodiments the inner diameter of the tubular portion can be in the range of 0.25 mm to 5 mm, about 1 mm to about 4 mm, about 1.2 mm to about 3 mm, or about 1.25 mm to about 2.75 mm. The lumen of the tubular portion can also be defined with regards to a cross-sectional area. Exemplary cross-sectional areas are in the range of about 0.05 mm² to about 20 mm², or about 0.75 mm² to about 12.5 mm².

The thickness of the wall of the tubular member can be determined by calculating half the difference of the outer diameter and the inner diameter. The wall thickness may be uniform around the circumference of the wall, or may be non-uniform. In exemplary embodiments the thickness of the wall of tubular portion can be about 0.025 mm or greater, about 0.05 mm or greater, about 0.075 mm or greater, or about 0.10 mm or greater, such as in the range of about 0.05 mm to about 2.5 mm, about 0.05 mm to about 0.5 mm, about 0.05 mm to about 0.25 mm, about 0.05 mm to about 0.20 mm, about 0.05 mm to about 0.15 mm, about 0.10 mm to about 0.25 mm, or about 0.15 mm to about 1. 5 mm.

For purposes of explaining aspects of the disclosure, and with reference to Figure 2, the tubular portion can be described as having a first lengthwise half **141,** shown as the (upper) semi-circular half, and a second lengthwise half **143,** shown as the (lower) semi-circular half. The first and second lengthwise halves are understood to extend between the proximal and distal ends running the length of the tubular portion. At least the first lengthwise half **141** can include a separation margin that can fracture when force is applied to the insertion tool (e.g., force applied to the tab of the tool, as discussed herein).

In one aspect, the separation margin can be in the form of a groove **150** (e.g., fissure, crevice, scored line, indentation) in the wall of the first lengthwise half **141.** The groove **150** can be of any desired shape, such as V-shaped, U-shaped, rectangular or square shaped. A V-shaped groove **150** in the outer wall of the first lengthwise half **141** is shown in Figure 2. The groove can be described in terms of its depth in the wall of the tubular portion. For example, in some embodiments the depth of the groove can be at least about 10% of the thickness of the wall where the groove is present. In some embodiments the depth of the groove can be in the range of about 10% to about 75% of the thickness of the wall, or about in the range of about 20% to about 60% of the thickness of the wall. Exemplary groove depths are in the range of about 0.025 mm to about 1 mm, about 0.05 mm to about 0.25 mm, or about 0.05 mm to about 0.15 mm. For example, for a wall thickness of about 0.5 mm, the groove may be a depth in the range of about 0.05 mm to about 0.375 mm, or for a wall thickness of about 0.075 mm, the groove may be a depth in the range of about 0.025 mm to about 0.070 mm. The desired depth of the groove can be determined by the wall thickness, the material used to make the tubular portion, the groove configuration, or combinations thereof.

In other aspects, the separation margin is a structural weakening in the wall of the tubular portion. The weakening can be due to a difference in the material of the tubular portion, or a difference in the physical property (e.g., molecular orientation) of the material at the separation margin as compared to another portion of the tubular member. Such a weakening can be introduced in the tubular member by a manufacturing process, such as extrusion processing. An extrusion process can induce a structural weakening along the separation margin by stretching the polymer composition used to make the tubular member. Stretching can be performed before extrusion, during extrusion, after extrusion, or combinations thereof. Stretching can induce coaxial alignment of the polymer molecules along the separation margin which can weaken the tubular member when force is applied in a direction perpendicular to the orientation of the polymer molecules.

The separation margin can be continuous along the first lengthwise half **141** (i.e., running from the proximal to distal end of the tubular portion), or non-continuous. If the separation margin is non-continuous along the first lengthwise half **141** it preferably includes few non-grooved interruptions to so the tubular member can still be separated in this half. Further, the separation margin can follow a linear path in the first lengthwise half **141,** or alternatively can follow a non-linear path along the length (e.g., the separation margin can include one or more curves, angles, etc.)

In addition to the separation margin along the first lengthwise half **141,** the tubular portion can optionally include one or more additional grooves in the first lengthwise half **141,** the second lengthwise half **143,** or both. If the tubular member includes one or more other (e.g., second, third, etc.) groove(s), the groove may or may not function as a separation margin. For example, the tubular portion may include a groove **152** in the second lengthwise half **143,** such as one positioned geometrically opposite to the groove **150** in the first lengthwise half **141.**

Upon application of force to the tubular member, groove **150** may fracture causing a split in the first lengthwise half **141** (e.g., due to force between the balloon catheter shaft and the inner surface of the tubular member adjacent to the groove **150).** However, the same force may not cause any fracturing of the tubular wall adjacent to groove **152** (if such a groove is present), and therefore the second lengthwise half **143** may stay intact when the insertion tool is removed from the catheter shaft. Fabrication of the tubular member can be facilitated by forming pairs of grooves in the tubular member, with one groove geometrically opposite the other.

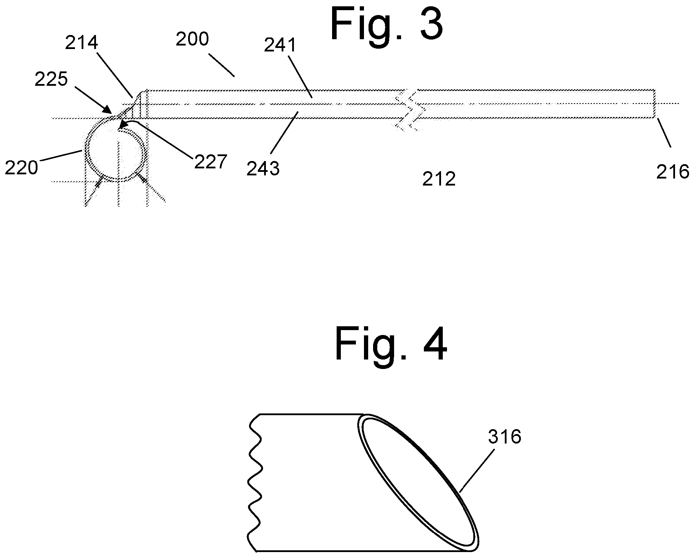

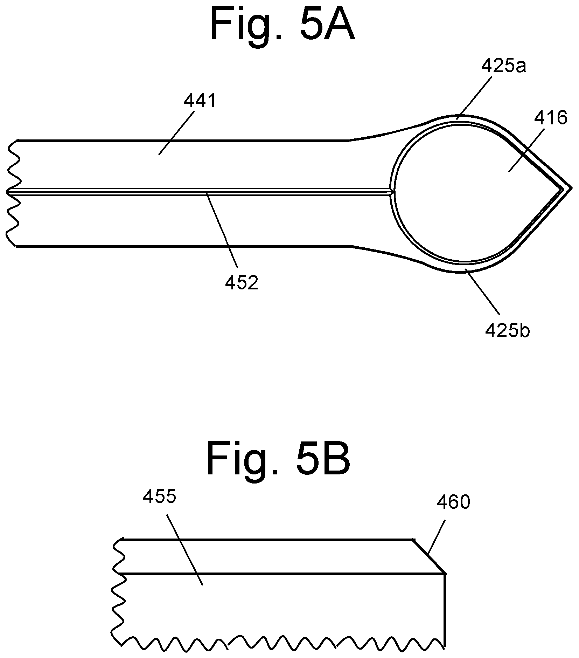

Referring now to Figure 3, embodiments of the insertion tool include those wherein the proximal and/or distal ends of the tubular portion are flush, as well as those wherein the proximal and/or distal ends of the tubular portion are not flush. An insertion tool **200** with a flush (flat) distal end **216** of the tubular member **212.** The end **214** of the tubular member **212** is shown having a tapered end, wherein tubular portion tapers from the first lengthwise half **241** to the second lengthwise **half 243.** Examples of non-flush configurations include tapered configurations and flared configurations. Figure 4 illustrates an embodiment of an end of an insertion tool with a tapered configuration **316,** which can be formed by cutting the distal end of a tubular article at an angle. An example of an end with a tapered and flared configuration **416** is shown in Figure 5a, which is a top view of an end portion of an insertion tool showing the first lengthwise half **441** with separation margin **452,** wherein the end **416** flares out. The flaring results in a widening of tubular portion at the end, as reflected by the increased width between points **425a** and **425b** as compared to the width (outer diameter) of the tubular portion that is adjacent to the end **416.**

An insertion tool that includes a flared proximal opening can allow easier insertion of a balloon through the end, and can minimize or prevent the removal of therapeutic agent from the balloon surface, wherein the removal may be otherwise caused by frictional forces. Flaring the distal opening will allow to advance the tool over the balloon without scraping off drug.

Embodiments of the disclosure also include those wherein the wall of the tubular portion is tapered. For example, with reference to Figure 5b, which shows a portion of a cross section of a tubular portion **455,** the wall **460** at the end is tapered.

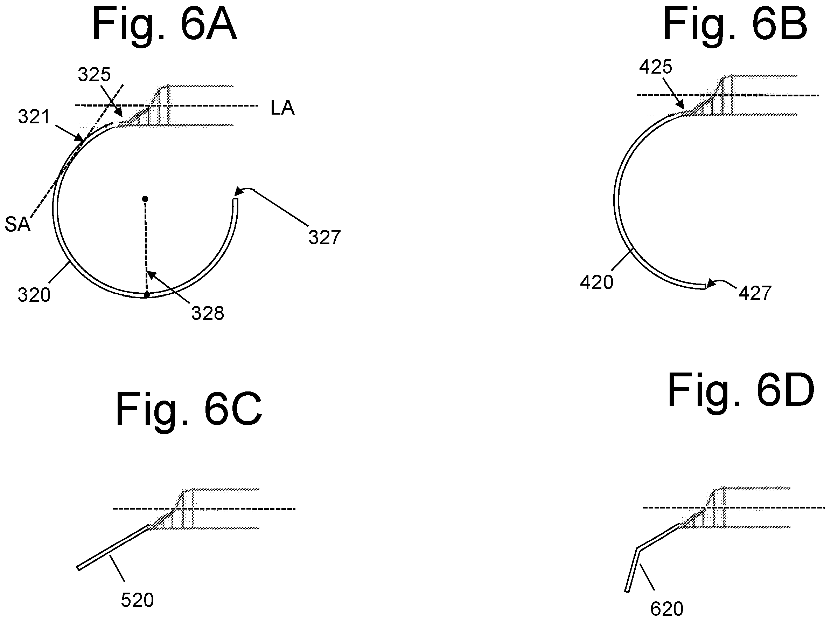

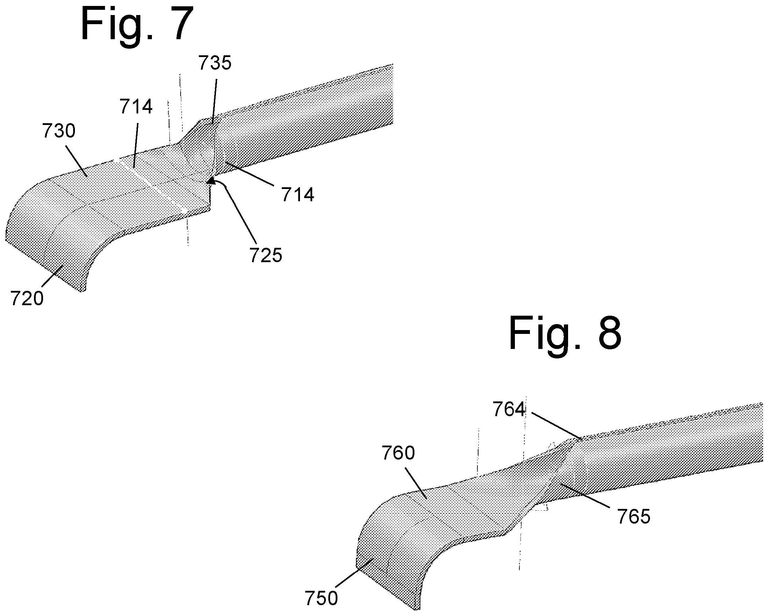

Embodiments of the disclosure include those wherein the insertion tool includes a tab at the proximal end of the tubular member, wherein the tab has a portion that extends from the second lengthwise half of the tubular portion, the tab portion at an angle skew to the lengthwise axis of the tubular portion. Examples of tabs having a portion that extends from the second lengthwise half of the tubular portion and skew to the lengthwise axis are shown in Figure 1 **(120),** Figure 3 **(220),** Figure 6A **(320),** Figure 6B **(420),** Figure 6C **(520),** Figure 6D **(620),** and Figure 7 **(720).**

In embodiments, the tab can include curved shape, such as shown in Figures 1, 3, 6A, and 6B which are side views of proximal end portions of insertion tools. With reference to Figure 6A, **321** points to a portion of the tab that is skew (dashed line SA representing the skew axis) to the lengthwise axis (dashed line LA) of the tubular member. It is understood that for a tab that include curved shape there can be multiple portions of the tab that are skew to the lengthwise axis of the tubular member.

A curved tab can be described various ways, such as by the length of the tab that follows the curvature. For example, this length can be represented in Figure 3, from point **225** to **227;** Figure 6A, from point **325** to **327;** and in Figure 6B, from point **425** to **427.** Exemplary tab lengths are in the range of about or about 5 mm to about 100 mm, or about 20 mm to about 40 mm.

A curved tab can also be described by the extent of its curve, expressed in degrees. For example, the curve of the tab in Figure 3 is approximately 360°, whereas the curve of the tab in Figure 6A is approximately 270°, and whereas the curve of the tab in Figure 6B is approximately 180°. Exemplary curves are in the range of approximately 15° to approximately 540°, or approximately 30° to approximately 360°, or preferably approximately 45° to approximately 270°.

A curved tab can also described by its radius. For example, the radius of tab in Figure 6A is represented by line **328.** The radius of the curved tab may stay the same along the length of the tab, or can change. In some embodiments the radius of the tab can decrease, such as by up to about 50% of the starting radius (e.g., the radius adjacent or at **point 235),** or up to about 25% of the starting radius. Exemplary curved tab radii are in the range of about 1 mm to about 20 mm, or about 3 mm to about 10 mm.

In embodiments of the disclosure as illustrated in Figures 6C and 6D, the tab (e.g., as viewed from a lengthwise cross-section of the insertion tool), can have one or more non-curved (e.g., straight) portions as represented by tab portions **520** and **620.**

Embodiments of the disclosure include those wherein the tab has a second portion that extends from the second lengthwise half of the tubular portion, the second tab portion parallel the lengthwise axis of the tubular portion. Figure 7 is an illustration of a tab that has a first portion **720** (i.e., a curved portion) that is at an angle skew to the lengthwise axis of the tubular portion, and a second portion **730** (i.e., a flat portion) that is parallel to the lengthwise axis of the tubular portion. Therefore, the second portion **730** will have a widthwise axis (e.g., dashed line **714)** that is perpendicular to the lengthwise axis of the tubular member, and lengthwise axis that is parallel to the lengthwise axis of the tubular member, whereas the first portion **720** will have a widthwise axis that is perpendicular to the lengthwise axis of the tubular member, and lengthwise axis that is skew to the lengthwise axis of the tubular member.

Further, aspects of the tab immediately adjacent to the proximal end of the tubular portion can be described. For example, the tab can have a distal portion **725** that is curved (the distal portion extending from the curve of the second lengthwise half of the proximal end of the tubular portion). As the tab extends proximally, it can flatten (widthwise) from the curved shape. Also as the tab extends proximally the width of the tab can widen from a narrower width adj acent to the proximal end of the tubular member. Therefore the width of the tab can increase in a distal to a proximal direction. In exemplary embodiments, the width of the tab can be in the range of about 0.5 mm to about 30 mm, or about 2 mm to about 3.5 mm. A notch **735** can be present in the first lengthwise half of the tubular member, at the proximal end.

Figure 8 is an illustration of another embodiment showing a tab having a first portion **750** (i.e., a curved portion) that can be at an angle skew to the lengthwise axis of the tubular portion, and a second portion **760** (i.e., a flat portion) that can be parallel to the lengthwise axis of the tubular portion. Figure 8 also shows that between the proximal end **764** of the tubular portion and the second portion **760** (i.e., a flat portion) can be a tapered transition portion **765.**

A tab that extends from the second lengthwise half of the tubular portion, and having a portion that can be at an angle skew to the lengthwise axis of the tubular portion can be formed by processing a flush end of a tubular member. For example, the following steps can be carried out to form a tab: (a) provide a tube having flush proximal and distal ends; (b) cut a slit partially through and perpendicular to the lengthwise axis of the tube to at least half the distance through the tube; (c) make a second cut into the tube between the proximal end and the slit, the cut made at an angle to meet the bottom of the slit cut (thereby forming a wedge-shaped cut in the upper half of the tube); force the cut proximal end downwards to flatten at least a portion of it (e.g., using heat forming).

In another embodiment, the insertion tool can include a split in the wall in the first half of the tubular portion. In this embodiment, during an insertion process the balloon catheter can be held within the inner diameter of a tubular member having such a configuration. After the insertion tool facilitates the insertion of the balloon catheter through the hemostatic valve, the tubular portion can be moved proximally so the tubular portion surrounds a portion of the balloon catheter shaft. Force can be then be applied to the tab so the first half of the tubular portion opens to the movement of the catheter shaft out of the inner diameter of the tubular portion. In particular, the force applied to the tab can cause the walls of the tubular portion on either side of the split to move apart, thereby providing a lengthwise gap that the catheter shaft can be moved through.

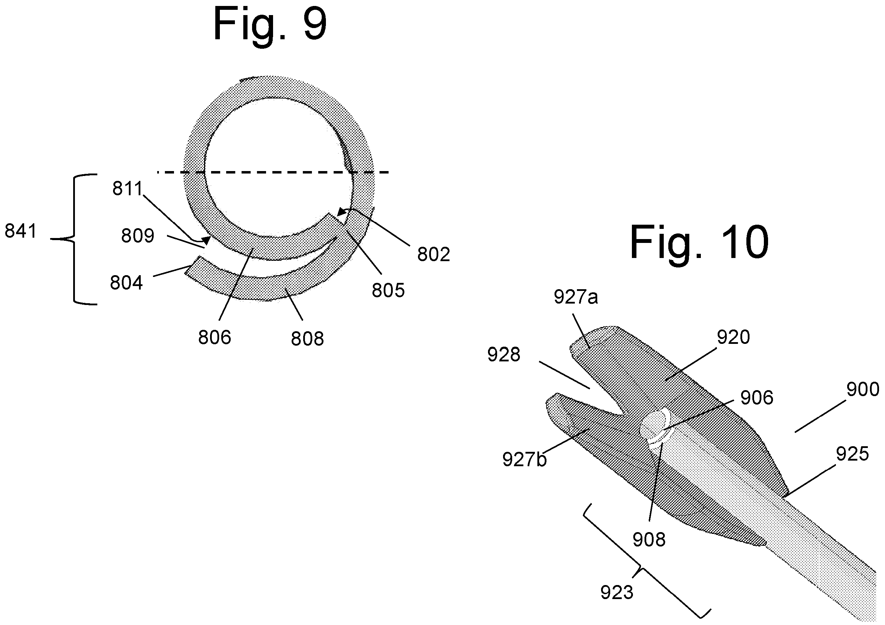

An exemplary embodiment of an insertion tool having a split in the wall in the first half of the tubular portion **841** is shown in Figure 9, which is a cross section view of the tubular portion from the distal end. The split in first half of the wall can provide first edge **802** and second edge **804,** which extend the length of the tubular portion. A point on the outer wall adjacent to first edge **802** and can contact a point **805** on the inner wall of tubular member in first half. As such, as viewed from the proximal end, the first half can include an inner overlapping portion of the wall **806,** and an outer overlapping portion of the wall **808.**

The circumferential distance between the first edge **802** and point **805** can form an inner diameter large enough to accommodate a balloon catheter of choice. For example, in these embodiments, the outer diameter of the tubular portion can be in the range of about 0.45 mm to about 10 mm, or about 1.5 mm to about 5 mm, and the inner diameter of the tubular portion can be in the range of 0.25 mm to 5 mm, or about 1 mm to about 4 mm. The dimensions/length of the outer overlapping portion of the wall **808** (between edge **804** and point **805)** can be described, such as in relation to other portions of the insertion tool. For example, the length **(804** - **805)** can be less than the circumference **802** - **805,** or a length in the range of about 5% to about half of the circumference (about 5% to 50% of **802** - **805).**

There also may be a gap **809** between the inner surface of the outer overlapping portion of the wall **808** (i.e., adjacent edge **804)** and the adjacent point **811** of the outer surface of the inner overlapping portion of the wall **806.** The gap **809** can be a distance in the range of a fraction of the wall thickness to many times the wall thickness, for example in the range of about 0 mm (no gap) to about 0.5 mm.

Figure 10 is a perspective view of the proximal portion of an insertion tool **900** having a split in the wall in the first half of the tubular portion, and a tab at the proximal end in the form of a solid article tab **920** comprising a concave surface that is attached (e.g., fastened or fixed) to an outer surface of the second lengthwise half of the tubular portion. Shown from the first half of the tubular portion are the inner overlapping portion of the wall **906,** and the outer overlapping portion of the wall **908.** The distance from the proximal end of the tubular portion to the distal end of the tab **925** (i.e., attachment length **923)** can be in the range of about 5 mm to about 25 mm. The tab **920** can include two or more proximal extension portions, for example proximal extension portions **927a** and **927b,** which can have a relatively flat shape. The extension portions can narrow from near the center of the tab to the proximal end of the tab (i.e., in a lengthwise direction). Further, the extension portions can have a thickness (i.e., in a radial direction) that is greater near the center of the tab than their thickness at the proximal end of the tab.

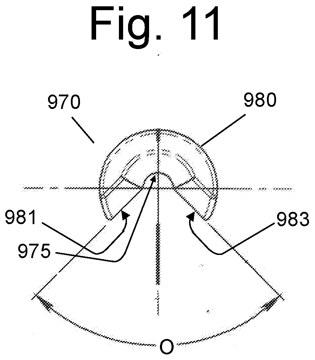

Figure 11 is a cross section that shows an embodiment of the solid article tab 970 with concave surface that can be fastened to an outer surface of the second lengthwise half of the tubular portion as viewed from its proximal end. Concave surface **975** of tab **970** is shown, and this surface can have a semicircular shape with an inner diameter that corresponds to the outer diameter of the second half of the proximal end of the tubular portion. The outer diameter of the second half of the proximal end of the tubular portion can be attached to the concave surface **975** of tab **970** though a suitable attachment material or member, such as an adhesive.

Figure 11 also shows that the outer surface **980** of the tab **970** can be curved (e.g., having a partially circular shape) as viewed from its proximal end. The outer surface can extend a desired distance as defined by degrees, such as a distance in the range of about 45° to about 300°, or about 180° to about 270°, such as measured between surfaces **981** and **983.** The opening **O,** or gap, between surfaces **981** and **983,** can also be defined, and can be in the range of about 60° to about 315°, or about 90° to about 180°. The solid article tab (e.g., **920, 970)** can also be defined by a thickness between its inner and outer surfaces (e.g., between concave surface **975** and outer surface **980),** with an exemplary thickness in the range of about 0.5 mm to about 5 mm, or about 1 mm to about 2 mm. In one mode of fabrication, an adhesive is applied between concave surface **975** and the outer surface of the second lengthwise half of the tubular portion (i.e., opposite the first lengthwise half including the split in the wall) in order to attach the solid article tab **970** to the tubular portion.

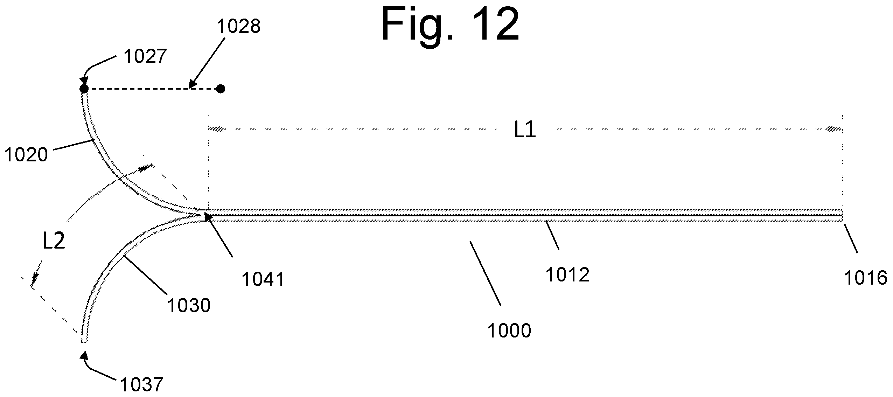

Referring to Figure 12, the inventive insertion tool includes two curved tabs at the proximal end of the insertion tool. The insertion tool **1000** is shown with a flush (flat) distal end **1016** of the tubular portion **1012.** Alternatively, the distal end can be tapered, such as according to a tapered configuration of other tool embodiments of the disclosure. The tubular portion **1012** extends a predetermined distance (L1) from the distal end **1016** to a point **1041** where tabs **1020** and **1030** begin. Point **1041** is a location of a split in the tubular member. At point **1041** one half of the tubular member forms a first tab **1020,** and the other half of the tubular member forms a second tab **1030.** Tabs **1020** and **1030,** as shown in the figure, are curved. Tabs **1020** and **1030** can terminate at proximal ends **1027** and **1037,** respectively.

Tabs **1020** and **1030** can follow any curved path, such as a circular or an elliptical path. Tabs **1020** and **1030** can also be described by the extent of its curve, expressed in degrees. For example, the curve of Tabs **1020** and **1030** in Figure 12 is approximately 90°, and exemplary tabs are in the range of approximately 45° to approximately 270°, for the inventive insertion tool, approximately 45° to approximately 180°. The curved tabs **1020** and **1030** can also described by their radii. For example, the radius of tab **1020** is represented by line **1028.** The radius of tab **1020** can be the same or different than tab **1030.** The radius of the curved tab may stay the same along the length of the tab, or can change. In this embodiment, exemplary curved tab radii are in the range of about 5 mm to about 35 mm, or, for the inventive insertion tool, about 10 mm to about 30 mm.

The insertion tool **1000** can also be described in terms of the length of the tubular portion **1012** and one or both tabs **1020** and **1030.** In embodiments, the length L1 of the tubular portion **1012** is greater than the length L2 one or both tabs **1020** and **1030,** and the length L1 of the tubular portion **1012** is two or more times, or three or more times greater than the length L2 of one or both tabs **1020** and **1030.** In exemplary the length L1 of the tubular portion **1012** is in the range of about 25 mm to about 150 mm, or about 75 mm to about 125 mm.

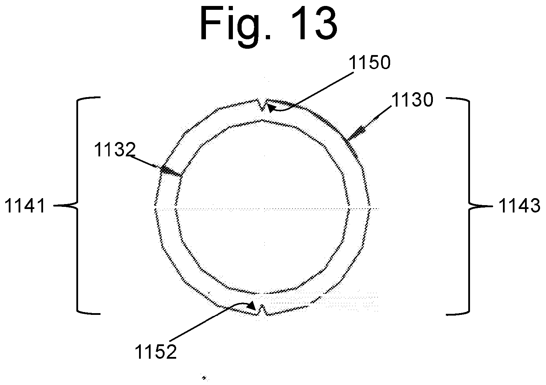

The insertion tool **1000** of Figure 12 can be prepared using a tubular portion having a configuration according to Figure 13 (wherein the tubular portion is similar to, or the same as, the one shown in Figure 2), which illustrates the tubular portion as viewed from the distal end. The tubular portion can have a first lengthwise half **1141** as the (left) semi-circular half, and a second lengthwise half **1143** as the (right) semi-circular half. The first and second lengthwise halves are understood to extend between the proximal and distal ends running the length of the tubular portion **1012.** The first and second lengthwise halves are defined by first separation margin **1150** (e.g., a V-shaped groove) and second separation margin **1152** (e.g., another V-shaped groove). Tabs **1020** and **1030** are formed as extensions of the first lengthwise half **1141** and second lengthwise half **1143** which are split at point **1041** and configured to angle away (e.g., as in a curved arrangement as shown) from the central axis of the tubular portion **1012.** Figure 13 also shows the outer surface **1130** and the inner surface of the **1132** tubular portion.

In some embodiments of the insertion tool the first separation margin **1150** and the second separation margin **1152** can be structural weakenings in the tubular portion **1012,** the weakenings representing a difference in the material of the tubular portion, or a difference in the physical property (e.g., molecular orientation) of the material at the separation margins. In some embodiments, the weakening can be introduced in the tubular member by the process of manufacture. As a non-limiting example, a structural weakening in the tubular portion can be intentionally induced through extrusion processing. An extrusion process can induce a structural weakening along the separation margin by stretching the polymer composition used to make the tubular member. Stretching can be performed before extrusion, during extrusion, after extrusion, or combinations thereof. Stretching can induce coaxial alignment of the polymer molecules along the separation margin which can weaken the tubular member when force is applied in a direction perpendicular to the orientation of the polymer molecules. This also can create a natural split at point **1041.**

Force can be applied to the insertion tool **1000** to cause separation of the first and second lengthwise halves **(1141** and **1143).** For example, a user can pull tabs **1020** and **1030** outwardly, away from the central axis of the tubular portion **1012.** This in turn can cause the tubular portion **1012** to fracture along first and second separation margins **(1150** and **1152).**

The embodiment of Figure 12 can include any dimension of overall length, outer diameter, inner diameter, or wall thickness as described herein; can include any tube configuration including distal end configuration as described herein; can be formed from any material or by any process as described herein; and can be used with any balloon catheter or hemostatic valve as described herein, or known in the art.

The tubular portion, tab, or both, can be formed by techniques such as extrusion, 3D printing, injection molding, compression molding, particulate leaching, solvent casting, thermoforming, or cutting. A combination of fabrication techniques can be used. Features of the insertion tool (e.g., separation margin, tab) can be formed during extrusion, molding, etc., or afterwards. Exemplary materials that can be used to fabricate a part of, or all of, the insertion tool include polymer-based materials such as fluorinated ethylene propylene (FEP); low and high density polyethylene (HDPE and LDPE), polytetrafluoroethylene (PTFE; Teflon); polyurethane; PEBAX, polyesteramide, polyimide, polyester, and polyamide (Nylon).

The material used to make portions of the insertion tool, such as the tubular portion, thereof can be transparent and able to transmit UV light. Various aliphatic polymers, including halogenated aliphatic polymers can provide good transmission of UV light. In some embodiments, a coating composition including a UV-activated crosslinker as described herein can be applied to the inner surface (inner diameter) of the tubular member of the insertion tool, and the tubular member can be irradiated with UV light which traverses the material of the tubular member and activates the crosslinker to form a durable coating.

All or part of the insertion tool can be fabricated to provide a visual or detectable distinction between the insertion tool and another part of the balloon catheter insertion system, such as the balloon catheter and/or the hemostatic valve. Components of the balloon catheter insertion system may be fabricated from similar materials, and therefore portions of the system components may be otherwise difficult to distinguish from each other when they are used together if a detection material is not used with one or more components of the system. For example, the detection material can be a colorant, material which reflects light (e.g., to increase the opacity of the insertion tool), a radioopaque material, a paramagnetic material, a vapor phase material, or a radioisotopic materials.

For example, a colorant or imaging agent can be provided at one or more portions along the length of the insertion tool and/or balloon catheter. The colorant or imaging agent can facilitate monitoring of the progress of insertion of the balloon catheter into a patient. The colorant(s) or imaging agent can provide a visual cue to the practitioner that indicates the spatial relationship of a part of the insertion tool with a part of the balloon catheter. Being able to visually determine the portions of the device can improve the insertion process.

A colorant or imaging agent can be used on or in a polymeric material used that is used to fabricate the insertion tool and/or balloon catheter. A colorant can also be used in a lubricious coating material (such as a polymeric hydrogel coating) that is optionally applied to a surface of insertion tool and/or balloon catheter.

Example of colorants include, but are not limited to, FD&C and D&C lakes, titanium dioxide, magnesium carbonate, talc, pyrogenic silica, iron oxides, channel black, insoluble dyes, natural colorants (such as riboflavin, carmine 40, curcumin, and annatto), dyes approved for ingestion by the U.S. Federal Drug Administration, or a combination of any of these. Colorants used in making coating dispersions for coating tablets, food, confectionery forms, agricultural seeds, and the like can be used in association with articles of the current disclosure.

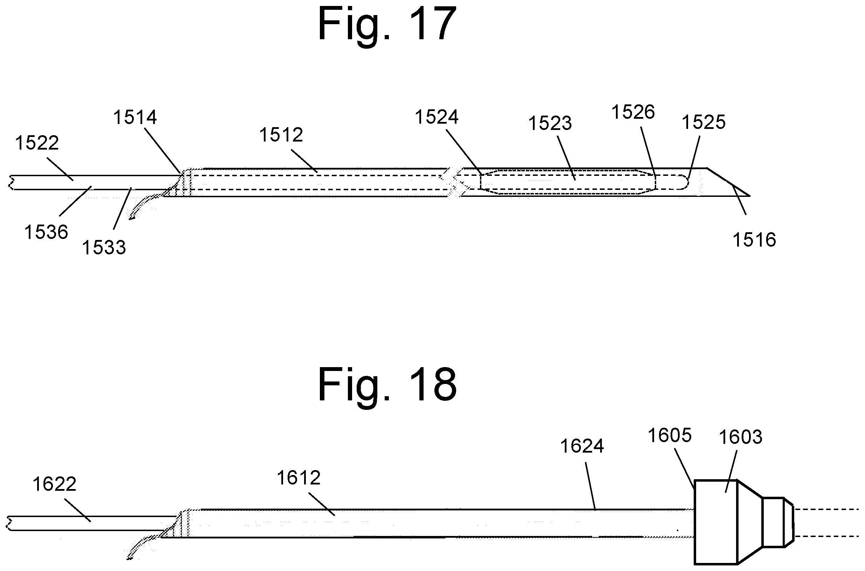

A colorant or imaging agent can be present on one or more portions of the insertion tool, the balloon catheter, or any other component that may be used in conjunction with the tool and catheter, in order to facilitate entry of the catheter into the body. For example, with reference to Figure 17, balloon portion **1523** is shown located within the tubular portion **1512** of an insertion tool. A first catheter marking **1536** and second catheter making **1533** can be located at points along the length of the catheter body **1522,** and visible to a user during the insertion process. Markings along the catheter body can positionally correspond to portion(s) of the catheter (e.g., points along the balloon portion **1523),** which may not be visible to the user. For example, the distance between second catheter making **1533** and the proximal end of the insertion tool **1514** can correspond to the distance between the distal tip of the balloon catheter **1525** and the distal end of the insertion tool **1516.** Monitoring the position of the second catheter making **1533** relative to the proximal end of the insertion tool **1514** can allow the user to understand when the distal tip **1525** emerges from the distal end **1516** during an insertion process. Likewise, the distance between the first catheter marking **1536** and the distal end **1516** (or the second catheter making **1533)** can allow the user to understand the location of the distal end of the balloon portion **1526** during insertion of the balloon catheter.

With reference to Figure 18, markings can also be present on the outer surface of the tubular member of the insertion tool **1612** and can be helpful for understanding the positioning of the distal end of the insertion tool (not shown) relative to portions of a hemostatic valve **1603** which the insertion tool **1612** is being passed through. For example, in use, the insertion tool **1612** through hemostatic valve **1603** to marking **1624,** which can indicate the distal end of the insertion tool has reached the distal end of the hemostatic valve. Following this, the body of the balloon catheter **1622** can be advanced to move the balloon catheter into the patient.

Alternatively, or in addition to the insertion tool marking, the tubular member of the insertion tool can include a stop member (not shown) that extends radially outwards from its surface, that meets a portion of the proximal end **1605** of the hemostatic valve, and which effectively prevents further distal movement of the insertion tool through the hemostatic valve. The stop member may be in the form of a raised circumferential lip or bulge from the outer surface of the tubular member, or any other structure that is able to contact the proximal portion of the hemostatic valve. The stop member can be formed as part of an extrusion process used to make the insertion tool, or can be added to the tubular member after it is formed, for example by adhering a plastic ring to a desired location on the outer surface of the tubular member.

In embodiments of the disclosure, any portion of any insertion tool/article, or any portions of catheter of the disclosure can have a coating, such as a hydrophilic lubricious coating. For example, hydrophilic polymeric base coatings can be applied to portions of the insertion tool/article, or any portions of catheter to impart lubricity and decrease loss of desired material (e.g., therapeutic agent from the balloon surface). In other embodiments, any portion of any insertion tool/article, or any portions of catheter of the disclosure can be associated with a low friction article such as a Teflon sleeve. In some embodiments, all or a portion of the inner diameter of the tubular member of the insertion tool is coated with a hydrophilic coating, or lined with lubricious low friction sleeve (e.g. PTFE and PTFE liners). In some embodiments, all or a portion of the outer surface of the balloon catheter is coated with a hydrophilic coating, or lined with lubricious low friction sleeve. Other materials for providing a lubricious low friction coating includes silicone oil, perfluorinated oils and waxes, optionally with covalently bonding, which imparts lower friction.

One class of hydrophilic polymers useful as polymeric materials for hydrophilic base coat formation can be synthetic hydrophilic polymers. Synthetic hydrophilic polymers that are biostable (i.e., that show no appreciable degradation *in vivo)* can be prepared from any suitable monomer including acrylic monomers, vinyl monomers, ether monomers, or combinations of any one or more of these types of monomers. Acrylic monomers include, for example, methacrylate, methyl methacrylate, hydroxyethyl methacrylate, hydroxyethyl acrylate, methacrylic acid, acrylic acid, glycerol acrylate, glycerol methacrylate, acrylamide, methacrylamide, dimethylacrylamide (DMA), and derivatives and/or mixtures of any of these. Vinyl monomers include, for example, vinyl acetate, vinylpyrrolidone, vinyl alcohol, and derivatives of any of these. Ether monomers include, for example, ethylene oxide, propylene oxide, butylene oxide, and derivatives of any of these. Examples of polymers that can be formed from these monomers include poly(acrylamide), poly(methacrylamide), poly(vinylpyrrolidone), poly(acrylic acid), poly(ethylene glycol), poly(vinyl alcohol), and poly(HEMA). Examples of hydrophilic copolymers include, for example, methyl vinyl ether/maleic anhydride copolymers and vinyl pyrrolidone/(meth)acrylamide copolymers. Mixtures of homopolymers and/or copolymers can be used.

Examples of some acrylamide-based polymers, such as poly(N,Ndimethylacrylamide-co-aminopropylmethacrylamide) and poly(acrylamide-co-N,Ndimethylaminopropylmeth-acrylamide) are described in example 2 of U.S. Patent No. 7,807,750 (Taton et al.)*.*

Other hydrophilic polymers that can be useful in the present disclosure are derivatives of acrylamide polymers with photoreactive groups. One such representative hydrophilic polymer can be the copolymerization of N-[3-(4-benzoylbenzamido)propyl]methacrylamide (Formula I) with N-(3-aminopropyl)methacrylamide (Formula II) to produce the polymer poly(N-3-aminopropyl)methacrylamide-co- N-[3-(4-benzoylbenzamido)propyl]methacrylamide (Formula III). The preparation of the polymer is disclosed in Example 1 of US Patent Publication 2007/0032882 (to Lodhi, et al.)*.*

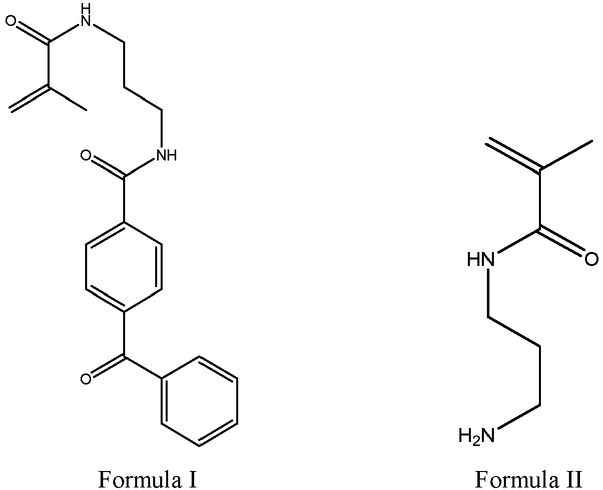

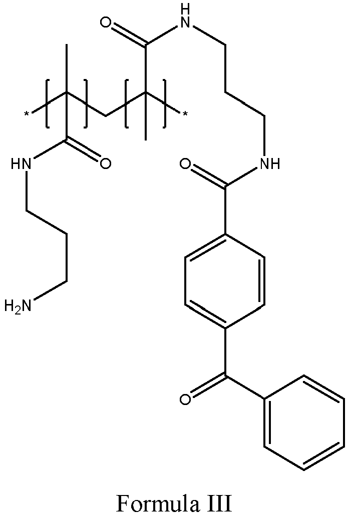

In some embodiments, the hydrophilic polymer can be a vinyl pyrrolidone polymer, or a vinyl pyrrolidone/(meth)acrylamide copolymer such as poly(vinylpyrrolidone-co-methacrylamide). If a PVP copolymer is used, it can be a copolymer of vinylpyrrolidone and a monomer selected from the group of acrylamide monomers. Exemplary acrylamide monomers include (meth)acrylamide and (meth)acrylamide derivatives, such as alkyl(meth)acrylamide, as exemplified by dimethylacrylamide, and aminoalkyl(meth)acrylamide, as exemplified by aminopropylmethacrylamide and dimethylaminopropylmethacrylamide. For example, poly(vinylpyrrolidone-co-N,N-dimethylaminopropylmethacrylamide) is described in example 2 of U.S. Patent No. 7,807,750 (Taton et al.)*.*

In one embodiment, the polymers and copolymers as described are derivatized with one or more photoactivatable group(s). Exemplary photoreactive groups that can be pendent from biostable hydrophilic polymer include aryl ketones, such as acetophenone, benzophenone, anthraquinone, anthrone, quinone, and anthrone-like heterocycles. Aryl ketones herein can specifically include diaryl ketones. Polymers herein can provide a hydrophilic polymer having a pendent activatable photogroup that can be applied to the expandable and collapsible structure, and can then treated with actinic radiation sufficient to activate the photogroups and cause covalent bonding to a target, such as the material of the expandable and collapsible structure. Use of photo-hydrophilic polymers can be used to provide a durable coating of a flexible hydrogel matrix, with the hydrophilic polymeric materials covalently bonded to the material of the expandable and collapsible structure.

A hydrophilic polymer having pendent photoreactive groups can be used to prepare the flexible hydrogel coating. Methods of preparing hydrophilic polymers having photoreactive groups are known in the art. For example, methods for the preparation of photo-PVP are described in U.S. Patent No. 5,414,075 (to Swan et al.)*.* Hydrophilic photo-polyacrylamide polymers such as poly(acrylamide-co-N-(3-(4-benzoylbenzamido)propyl) methacylamide)"Photo PA," and derivatives thereof can be used to form hydrophilic base coats on articles in exemplary embodiments of the present disclosure. Methods for the preparation of photo-polyacrylamide are described in U.S. Patent No. 6,007,833 (to Chudzik et al.)*.*

Other embodiments of hydrophilic base coats include derivatives of photo-polyacrylamide polymers incorporating additional reactive moieties. Some exemplary reactive moieties include N-oxysuccinimide and glycidyl methacrylate. Representative photo-polyacrylamide derivatives incorporating additional reactive moieties include poly(acrylamide-co-maleic-6-aminocaproic acid-N-oxysuccinimide-co-N-(3-(4-benzoylbenzamido)propyl) methacrylamide) and poly(acrylamide-co-(3-(4-benzoylbenzamido)propyl)methacrylamide)-co-glycidylmethacrylate. Additional photo-polyacrylamide polymers incorporating reactive moieties are described in US Patent Nos. 6,465,178 (to Chappa, et al.), 6,762,019 (to Swan, et al.) and 7,309,593 (to Ofstead, et al.)*.*

Other embodiments of exemplary hydrophilic base coats that include derivatives of photo-polyacrylamide polymers incorporating additional reactive moieties can be found in U.S. Patent No. 6,514,734 (to Clapper, et al.).

In yet other embodiments, the hydrophilic base coat can include derivatives of photo-polyacrylamide polymers incorporating charged moieties. Charged moieties include both positively and negatively charged species. Exemplary charged species include, but are not limited to, sulfonates, phosphates and quaternary amine derivatives. Some examples include the negatively charged species N-acetylated poly(acrylamide-co-sodium-2-acrylamido-2-methylpropanesulfonate-co-N-(3-(4-benzoylbenzamido)propyl)methacrylamide)-co-methoxy poly(ethylene glycol) monomethacrylate. Other negatively charged species that can be incorporated into the hydrophilic base coat are described in US Patent No. 4,973,493 (to Guire et al.). Positively charged species can include poly(acrylamide-co-N-(3-(4-benzoylbenzamido) propyl)methacrylamide)-co-(3-(methacryloylamino)propyl)trimethylammonium chloride. Other positively charged species that can be incorporated into a hydrophilic base coat are described in US Patent No. 5,858,653 (to Duran et al.)*.*

In another embodiment, the polymers and copolymers as described are derivatized with one or more polymerizable group(s). Polymers with pendent polymerizable groups are commonly referred to as macromers. The polymerizable group(s) can be present at the terminal portions (ends) of the polymeric strand or can be present along the length of the polymer. In one embodiment polymerizable groups are located randomly along the length of the polymer.

Exemplary hydrophilic polymer coatings can be prepared using polymer grafting techniques. Polymer grafting techniques can include applying a nonpolymeric grafting agent and monomers to a substrate surface then causing polymerization of the monomers on the substrate surface upon appropriate activation (for example, but not limited to, UV radiation) of the grafting agent. Grafting methods producing hydrophilic polymeric surfaces are exemplified in U.S. Pat. Nos. 7,348,055; 7,736,689 and 8,039,524 (all to Chappa et al.)*.*

Alternatively, a coating composition can include thermally-reactive polymers (e.g., a hydrophilic polymer with pendent thermally reactive peroxide groups), such as described in U.S. Patent No. 7,807,750 (Taton et al.)*.* In exemplary embodiments a coating composition with a thermally reactive polymer is applied to the inner surface of the tubular member (inner diameter) and heated to cause chemical reaction of the activated pendent groups to the material of the tubular member and bonding of the polymer.

Optionally, a coating on an article of the current disclosure can include a crosslinking agent. A crosslinking agent can promote the association of polymers in a coating, or the bonding of polymers to a coated surface. The choice of a particular crosslinking agent can depend on the ingredients of the coating composition.

Suitable crosslinking agents can include two or more activatable groups, which can react with the polymers in the composition. Suitable activatable groups can include photoreactive groups as described herein, like aryl ketones, such as acetophenone, benzophenone, anthraquinone, anthrone, quinone, and anthrone-like heterocycles. A crosslinking agent including a photoreactive group can be referred to as a photo-crosslinker or photoactivatable crosslinking agent. The photoactivatable crosslinking agent can be ionic, and can have good solubility in an aqueous composition. Thus, in some embodiments, at least one ionic photoactivatable crosslinking agent can be used to form a coating. The ionic crosslinking agent can include an acidic group or salt thereof, such as selected from sulfonic acids, carboxylic acids, phosphonic acids, salts thereof, and the like. Exemplary counter ions include alkali, alkaline earths metals, ammonium, protonated amines, and the like.

Exemplary ionic photoactivatable crosslinking agents include 4,5-bis(4-benzoylphenyl-methyleneoxy) benzene-1,3-disulfonic acid or salt; 2,5-bis(4-benzoylphenylmethyleneoxy) benzene-1,4-disulfonic acid or salt; 2,5-bis(4-benzoylmethyleneoxy)benzene-1-sulfonic acid or salt; N,N-bis[2-(4-benzoylbenzyloxy)ethyl]-2-aminoethanesulfonic acid or salt, and the like. See U.S. Patent Nos. 6,077,698 (Swan et al.), 6,278,018 (Swan), 6,603,040 (Swan) and 7,138,541 (Swan).

Other exemplary ionic photoactivatable crosslinking agents include ethylenebis(4-benzoylbenzyldimethylammonium) dibromide and hexamethylenebis(4-benzoylbenzyl dimethylammonium) dibromide and the like. See U.S. Patent No. 5,714,360 (Swan et al.)*.*

In yet other embodiments, restrained multifunctional reagents with photoactivable crosslinking groups can be used. In some examples these restrained multifunctional reagents include tetrakis (4-benzoylbenzyl ether) of pentaerthyritol and the tetrakis (4-benzoylbenzoate ester) of pentaerthyritol. See U.S. Patent Nos. 5,414,075 (Swan et al.) and 5,637,460 (Swan et al.)*.*

Additional crosslinking agents can include those having formula Photo1-LG-Photo2, wherein Photo1 and Photo2 independently represent at least one photoreactive group and LG represents a linking group comprising at least one silicon or at least one phosphorus atom, wherein the degradable linking agent comprises a covalent linkage between at least one photoreactive group and the linking group, wherein the covalent linkage between at least one photoreactive group and the linking group is interrupted by at least one heteroatom. See U.S. Patent No. 8,889,760 (Kurdyumov, et al.)*.* Further crosslinking agents can include those having a core molecule with one or more charged groups and one or more photoreactive groups covalently attached to the core molecule by one or more degradable linkers. See U.S. Publ. Pat. App. No. 2011/0144373 (Swan, et al.)*.*

In some embodiments, the first and/or second crosslinking agent can have a molecular weight of less than about 1500 kDa. In some embodiments the crosslinking agent can have a molecular weight of less than about 1200, 1100, 1000, 900, 800, 700, 600, 500, or 400.

In some embodiments, at least one of the first and second crosslinking agents comprising a linking agent having formula Photo1-LG-Photo2, wherein Photo1 and Photo2, independently represent at least one photoreactive group and LG represents a linking group comprising at least one silicon or at least one phosphorus atom, there is a covalent linkage between at least one photoreactive group and the linking group, wherein the covalent linkage between at least one photoreactive group and the linking group is interrupted by at least one heteroatom.

In some embodiments, at least one of the first and second crosslinking agents comprising a linking agent having a formula selected from: 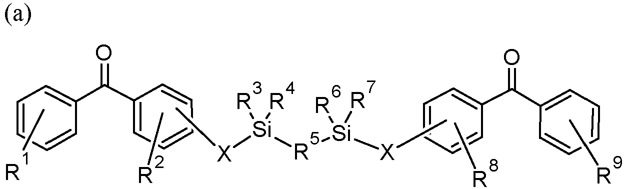 wherein R1, R2, R8 and R9 are any substitution; R3, R4, R6 and R7 are alkyl, aryl, or a combination thereof; R5 is any substitution; and each X, independently, is O, N, Se, S, or alkyl, or a combination thereof; 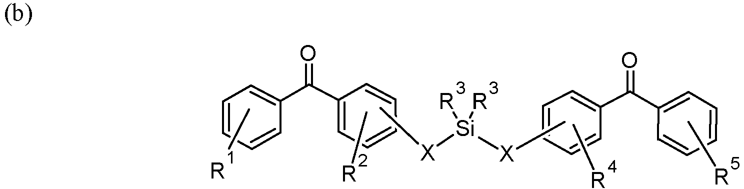 wherein R1 and R5 are any substitution; R2 and R4 can be any substitution, except OH; R3 can be alkyl, aryl, or a combination thereof; and X, independently, are O, N, Se, S, alkylene, or a combination thereof; 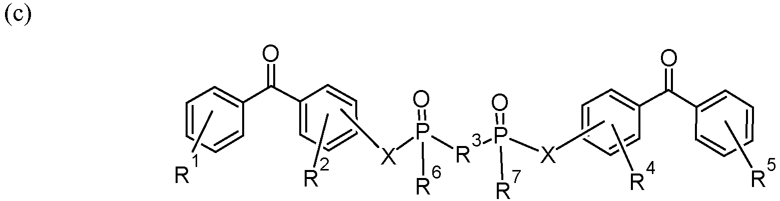 wherein R1, R2, R4 and R5 are any substitution; R3 is any substitution; R6 and R7 are alkyl, aryl, or a combination thereof; and each X can independently be O, N, Se, S, alkylene, or a combination thereof; and 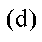 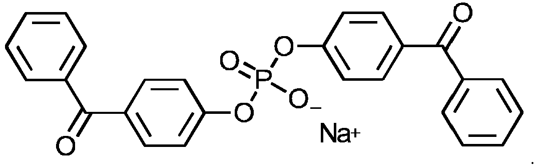 In a particular embodiment, the crosslinking agent can be bis(4-benzoylphenyl) phosphate.

In some embodiments, the photoactivatable crosslinking agent can be ionic, and can have good solubility in an aqueous composition, such as the first and/or second coating composition. Thus, in some embodiments, at least one ionic photoactivatable crosslinking agent is used to form the coating. In some cases, an ionic photoactivatable crosslinking agent can crosslink the polymers within the second coating layer which can also improve the durability of the coating.

Any suitable ionic photoactivatable crosslinking agent can be used. In some embodiments, the ionic photoactivatable crosslinking agent is a compound of formula I: X1-Y--X2 where Y is a radical containing at least one acidic group, basic group, or a salt of an acidic group or basic group. X1 and X2 are each independently a radical containing a latent photoreactive group. The photoreactive groups can be the same as those described herein. Spacers can also be part of X1 or X2 along with the latent photoreactive group. In some embodiments, the latent photoreactive group includes an aryl ketone or a quinone.

The radical Y in formula I provides the desired water solubility for the ionic photoactivatable crosslinking agent. The water solubility (at room temperature and optimal pH) is at least about 0.05 mg/ml. In some embodiments, the solubility is about 0.1 to about 10 mg/ml or about 1 to about 5 mg/ml.

In some embodiments of formula **I,** Y is a radical containing at least one acidic group or salt thereof. Such a photoactivatable crosslinking agent can be anionic depending upon the pH of the coating composition. Suitable acidic groups include, for example, sulfonic acids, carboxylic acids, phosphonic acids, and the like. Suitable salts of such groups include, for example, sulfonate, carboxylate, and phosphate salts. In some embodiments, the ionic crosslinking agent includes a sulfonic acid or sulfonate group. Suitable counter ions include alkali, alkaline earths metals, ammonium, protonated amines, and the like.

For example, a compound of formula I can have a radical Y that contains a sulfonic acid or sulfonate group; X1 and X2 can contain photoreactive groups such as aryl ketones. Such compounds include 4,5-bis(4-benzoylphenylmethyleneoxy) benzene-1,3-disulfonic acid or salt; 2,5-bis(4-benzoylphenylmethyleneoxy)benzene-1,4-disulfonic acid or salt; 2,5-bis(4-benzoylmethyleneoxy)benzene-1-sulfonic acid or salt; N,N-bis[2-(4-benzoylbenzyloxy)ethyl]-2-aminoethanesulfonic acid or salt, and the like. See U.S. Pat. No. 6,278,018 (to Swan). The counter ion of the salt can be, for example, ammonium or an alkali metal such as sodium, potassium, or lithium.

In other embodiments of formula I, Y can be a radical that contains a basic group or a salt thereof. Such Y radicals can include, for example, an ammonium, a phosphonium, or a sulfonium group. The group can be neutral or positively charged, depending upon the pH of the coating composition. In some embodiments, the radical Y includes an ammonium group. Suitable counter ions include, for example, carboxylates, halides, sulfate, and phosphate. For example, compounds of formula I can have a Y radical that contains an ammonium group; X1 and X2 can contain photoreactive groups that include aryl ketones. Such photoactivatable crosslinking agents include ethylenebis(4-benzoylbenzyldimethylammonium) salt; hexamethylenebis (4-benzoylbenzyldimethylammonium) salt; 1,4-bis(4-benzoylbenzyl)-1,4-dimethylpiperazinediium) salt, bis(4-benzoylbenzyl) hexamethylenetetraminediium salt, bis[2-(4-benzoylbenzyldimethylammonio)ethyl]-4-benzoylbenzylmethylammonium salt; 4,4-bis(4-benzoylbenzyl)morpholinium salt; ethylenebis[(2-(4-benzoylbenzyldimethylammonio)ethyl)-4-benzoylbenzylmethylammonium] salt; and 1,1,4,4-tetrakis(4-benzoylbenzyl)piperzinediium salt. See U.S. Pat. No. 5,714,360 (to Swan et al.)*.* The counter ion is typically a carboxylate ion or a halide. On one embodiment, the halide is bromide.

In other embodiments, the ionic photoactivatable crosslinking agent can be a compound having the formula: 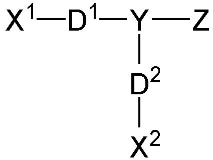 wherein X1 includes a first photoreactive group; X2 includes a second photoreactive group; Y includes a core molecule; Z includes at least one charged group; D1 includes a first degradable linker; and D2 includes a second degradable linker. Additional exemplary degradable ionic photoactivatable crosslinking agents are described in US Patent Application Publication US 2011/0144373 (Swan et al.*,* "Water Soluble Degradable Crosslinker").

In some aspects a non-ionic photoactivatable crosslinking agent can be used. In one embodiment, the non-ionic photoactivatable crosslinking agent has the formula XR1R2R3R4, where X is a chemical backbone, and R1, R2, R3, and R4 are radicals that include a latent photoreactive group. Exemplary non-ionic crosslinking agents are described, for example, in U.S. Pat. Nos. 5,414,075 and 5,637,460 (Swan et al.*,* "Restrained Multifunctional Reagent for Surface Modification"). Chemically, the first and second photoreactive groups, and respective spacers, can be the same or different.

In other embodiments, the non-ionic photoactivatable crosslinking agent can be represented by the formula:

PG2-LE2-X-LE1-PG1

wherein PG1 and PG2 include, independently, one or more photoreactive groups, for example, an aryl ketone photoreactive group, including, but not limited to, aryl ketones such as acetophenone, benzophenone, anthraquinone, anthrone, anthrone-like heterocycles, their substituted derivatives or a combination thereof; LE1 and LE2 are, independently, linking elements, including, for example, segments that include urea, carbamate, or a combination thereof; and X represents a core molecule, which can be either polymeric or non-polymeric, including, but not limited to a hydrocarbon, including a hydrocarbon that is linear, branched, cyclic, or a combination thereof; aromatic, non-aromatic, or a combination thereof; monocyclic, polycyclic, carbocyclic, heterocyclic, or a combination thereof; benzene or a derivative thereof; or a combination thereof. Other non-ionic crosslinking agents are described, for example, inPubl. No. US 2012/0149934(to Kurdyumov, "Photocrosslinker").

Further embodiments of non-ionic photoactivatable crosslinking agents can include, for example, those described in US Pat. Publication 2013/0143056 (Swan et al.*,* "Photo-Vinyl Linking Agents"). Exemplary crosslinking agents can include non-ionic photoactivatable crosslinking agents having the general formula R1 - X - R2, wherein R1 is a radical comprising a vinyl group, X is a radical comprising from about one to about twenty carbon atoms, and R2 is a radical comprising a photoreactive group.

A single photoactivatable crosslinking agent or any combination of photoactivatable crosslinking agents can be used in forming a coating. In some embodiments, at least one nonionic crosslinking agent such as tetrakis(4-benzoylbenzyl ether) of pentaerythritol can be used with at least one ionic crosslinking agent. For example, at least one non-ionic photoactivatable crosslinking agent can be used with at least one cationic photoactivatable crosslinking agent such as an ethylenebis(4-benzoylbenzyldimethylammonium) salt or at least one anionic photoactivatable crosslinking agent such as 4,5-bis(4-benzoyl-phenylmethyleneoxy) benzene-1,3-disulfonic acid or salt. In another example, at least one nonionic crosslinking agent can be used with at least one cationic crosslinking agent and at least one anionic crosslinking agent. In yet another example, a least one cationic crosslinking agent can be used with at least one anionic crosslinking agent but without a non-ionic crosslinking agent.

An exemplary crosslinking agent is disodium 4,5-bis[(4-benzoylbenzyl)oxy]-1,3-benzenedisulfonate (DBDS). This reagent can be prepared by combining 4,5-Dihydroxylbenzyl-1,3-disulfonate (CHBDS) with 4-bromomethylbenzophenone (BMBP) in THF and sodium hydroxide, then refluxing and cooling the mixture followed by purification and recrystallization (also as described in U.S. Pat. No. 5,714,360).

Further crosslinking agents can include the crosslinking agents described in US 8,487,137 (to Guire et al.) and U.S. Pat. No. 7,772,393 (to Guire et al.).

In some embodiments, crosslinking agents can include boron-containing linking agents including, but not limited to, the boron-containing linking agents disclosed in U.S. Patent No. 9,410,044 (to Kurdyumov). By way of example, linking agents can include borate, borazine, or boronate groups and coatings and devices that incorporate such linking agents, along with related methods. In an embodiment, the linking agent includes a compound having the structure (I): 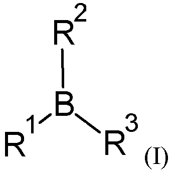 wherein R1 is a radical comprising a photoreactive group; R2 is selected from OH and a radical comprising a photoreactive group, an alkyl group and an aryl group; and R3 is selected from OH and a radical comprising a photoreactive group. In some embodiments the bonds B-R1, B-R2 and B-R3 can be chosen independently to be interrupted by a heteroatom, such as O, N, S, or mixtures thereof.

Additional agents for use with embodiments herein can include stilbene-based reactive compounds including, but not limited to, those disclosed in U.S. Pat. No. 8,487,137, entitled "Stilbene-Based Reactive Compounds, Polymeric Matrices Formed Therefrom, and Articles Visualizable by Fluorescence" by Kurdyumov et al.*.*

Additional photoreactive agents, crosslinking agents, hydrophilic coatings, and associated reagents are disclosed in U.S. Pat. No. 8,513,320 (to Rooijmans et al.); 8,809,411 (to Rooijmans); and 2010/0198168 (to Rooijmans).

Natural polymers can also be used to form a hydrophilic base coat. Natural polymers include polysaccharides, for example, polydextrans, carboxymethylcellulose, and hydroxymethylcellulose; glycosaminoglycans, for example, hyaluronic acid; polypeptides, for example, soluble proteins such as collagen, albumin, and avidin; and combinations of these natural polymers. Combinations of natural and synthetic polymers can also be used.

In some instances a tie layer can be used to form a hydrophilic base layer. In yet other instances the tie layer can be added to a hydrophilic base layer. The tie layer can act to increase the adhesion of the hydrophilic base layer to a substrate. In other embodiments, the tie layer can act to increase adhesion of a hydrophobic active agent to a hydrophilic base layer. Exemplary ties layers include, but are not limited to silane, butadiene, polyurethane and parylene. Silane tie layers are described in US Patent Publication 2012/0148852 (to Jelle, et al.)*.*

In exemplary embodiments, the hydrophilic base layer can include tannic acid, polydopamine or other catechol containing materials.

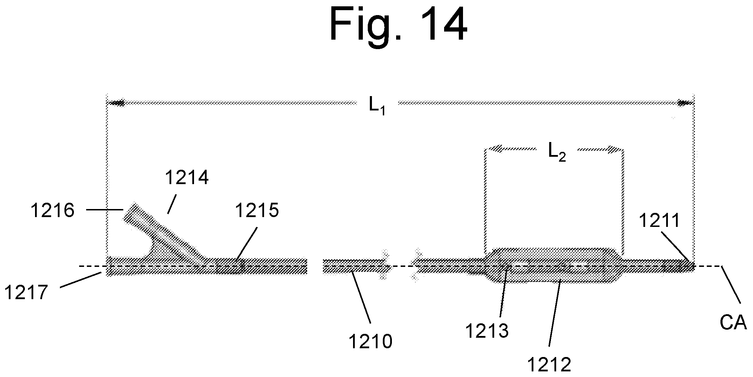

The insertion tool can be used in a system with a balloon catheter. Balloon catheters are commonly used in angioplasty procedures for the treatment of arteries that are diseased. Balloon angioplasty generally involves the dilation or reopening of blocked intraluminal channels. Balloon catheter constructions are well known in the art and are described in various documents, for example, U.S. Patent Nos. 4,195,637, 5,041,089, 5,087,246, 5,318,587, 5,382,234, 5,571,089, 5,776,101, 5,807,331, 5,882,336, 6,394,995, 6,517,515, 6,623,504, 6,896,842, 7,163,523, and 8,951,545. With reference to Figure 14, a balloon catheter (portions thereof not drawn to scale) generally includes four portions: the balloon **1212,** catheter shaft **1210,** guidewire (not shown), and manifold **1214.** The inflatable balloon **1212** is typically attached to a distal section of the flexible catheter shaft **2010,** and catheter shaft **2010** can include one or more aperture(s) **1213** that allow the balloon to be inflated, such as with a liquid. The catheter shaft can extend distally a short length to a distal tip **1211.** At the proximal end of the catheter shaft, there is typically the manifold **1214** which is configured to remain outside the patient and includes a guide wire port **1217** and an inflation fluid port **1216.** At the manifold end, placement of the catheter can be facilitated using a guidewire. Guidewires are small and maneuverable and can facilitate movement of the balloon catheter into the body through the insertion tool and the hemostatic valve. In some arrangements, the balloon and catheter is fixed to the guidewire, which can be moved together with the guidewire. In other arrangements, the balloon and catheter are not fixed to the guidewire and can be moved in relation to one another.

The catheter body is typically flexible so that it can navigate through the arterial system when introduced into a subject. The catheter can include a more rigid portion **1215** immediately distal to the inflation port **2014.** When in a straightened configuration (i.e., when the catheter body is straightened along a linear path), the catheter can have an axis "catheter axis" CA. The length (L₁) of balloon catheters can vary; standard lengths being in the range of about 50 cm to about 150 cm.

The balloon portion of the balloon catheter can be of various lengths (L₂) and a particular length can be used based on diagnosis of a patient and the size of the arterial area to be treated. Exemplary balloon lengths are in the range of about 20 mm to about 300 mm, about 25 mm to about 250 mm, or about 30 mm to about 160 mm, with "shorter" lengths being in the range of about 20 mm to about 60 mm, or about 30 mm to about 50 mm, with "longer" lengths being in the range of about 80 mm to about 300 mm, or about 100 mm to about 250 mm.

The balloon of the balloon catheter can also be of various diameters and a particular diameter can be used based on diagnosis of a patient and the relative diameter of the artery at the site to be treated. Balloon diameters are measured in an inflated state, and exemplary diameters are in the range of about 0.5 mm to about 12 mm, or about 1 mm to about 8 mm, or about 2 mm to about 7mm. Exemplary balloon diameters are about 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, and 12.0 mm.

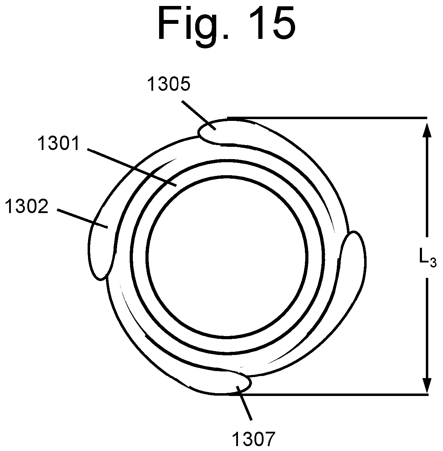

With reference to Figure 15 which shows a cross sectional view from the distal end of a balloon portion, prior to inflation the balloon can be folded to a compacted configuration for delivery to the target site. The balloon can be folded around the catheter body **1301** which in turn reduces the radial profile of the balloon catheter and facilitates movement through the insertion tool and into the body. A balloon folding process may involve creating "arms" **1302** of the balloon material and folding these arms inward (towards the catheter axis) to compact the balloon material. Depending on the balloon and the folding process in a deflated folded configuration the balloon can have two or more arms, such as three, four, five, six, seven, eight, nine, ten, eleven, or twelve folded arms.

In a folded configuration, the balloon portion can have a "maximum crossing profile," which is the maximum diameter found between the distal end of the manifold **(1215,** Figure 14) and the distal end (tip) of the catheter **(1211,** Figure 14). For example, the maximum crossing profile may occur at one or more points along the length of the folded balloon portion, along a continuous portion of the length, along most of the length, or along all of the length. The maximum crossing profile may also occur at the point where the balloon meets (e.g., is bonded or adhered to) the catheter shaft (see points **1524** and **1526** of Figure 17).

In an embodiment, and as shown in Figure 15, the folded balloon has a maximum crossing profile of length L₃. For example, length L₃ may be the diameter of the folded balloon between the outer surface of a distal end **1305** of folded balloon arm and distal end **1307** of radially opposite folded balloon arm. In some embodiments, the maximum crossing profile is about 2.20 mm or less, about 2.15 mm or less, about 2.10 mm or less, about 2.05 mm or less, about 2.00 mm or less, about 1.95 mm or less, about 1.90 mm or less, or about 1.85 mm. In some embodiments, the maximum crossing profile is in the range of about 1.60 mm to about 2.20 mm, about 1.65 mm to about 2.10 mm, or about 1.70 mm to about 2.00 mm, or about 1.75 mm to about 1.90 mm. In some embodiments, the maximum crossing profile is in the range of about 1.70 mm to about 2.00 mm, or about 1.75 mm to about 1.90 mm, and the balloon length is about 100 mm or less (e.g., about 20 mm to about 100 mm), or about 60 mm or less (e.g., about 20 mm to about 100 mm).

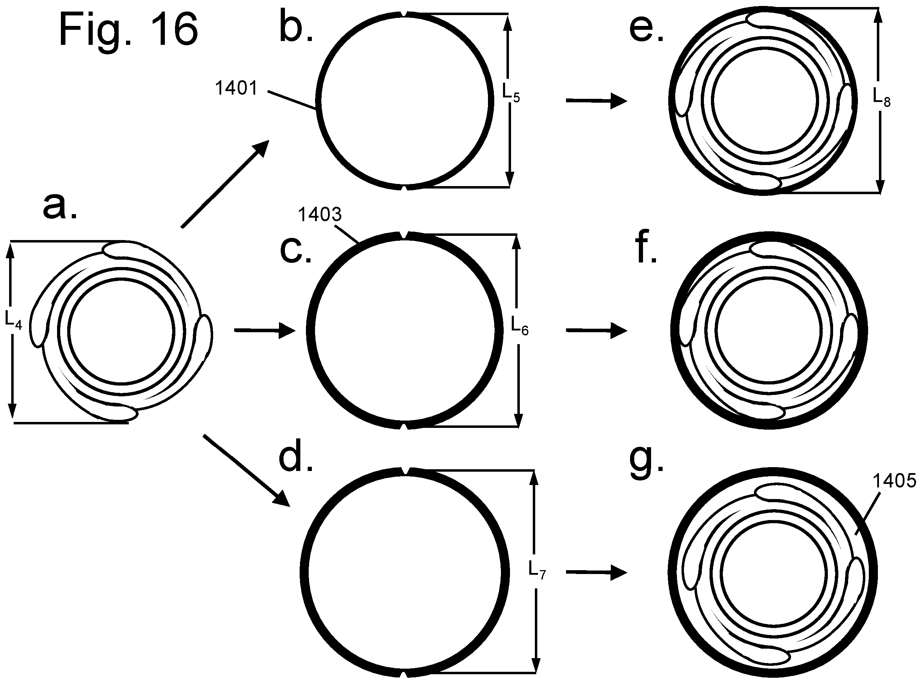

Figures 16a - 16g illustrate various embodiments of the loading of a folded balloon catheter into an insertion tool, as reflected by cross sectional views from the distal end of the balloon portion and insertion tool. Figure 16a is a folded balloon portion having a maximum crossing profile "L₄," such as in the range of about 1.70 mm to about 2.00 mm, or about 1.75 mm to about 1.90 mm. Figure 16b is a cross section of an insertion tool that has an inner dimeter of length L₅ that is not greater than the maximum crossing profile L₄ of the folded balloon. In some cases L₅ is less than L₄, for example L₅ is in the range of about 90% to about 99%, or about 95% to about 99%, of the length L₄. In these embodiments, the outer surface of the folded balloon will exert force upon the inner wall of the insertion tool, which can cause radial expansion of the insertion tool (e.g., deformation of the wall), further compression of the folded balloon, or both. For example, Figure 16e shows an insertion tool (from Figure 16b) loaded with a folded balloon causing radial expansion of the diameter of the insertion tool. In cases where L₅ is equal to or less than L₄, the wall **1401** of the insertion tool can be relatively thin, such as about less than 0.075 mm, less than 0.06 mm, or less than 0.05 mm, such as in the range of about 0.025 mm to about 0.06 mm, or about 0.025 mm to about 0.05 mm.

Figure 16c is a cross section of an insertion tool that has an inner dimeter of length L₆ that is at least the maximum crossing profile L₄ of the folded balloon, or slightly greater than L₄. In these embodiments, the folded balloon will fit in the inner diameter of the insertion tool with minimal or no open space between the inner wall of the insertion tool and the outer surface of folded balloon. This can be beneficial during insertion of the balloon catheter as it can minimize or prevent backflow of blood, which in turn improves safety of the procedure and can also maintain desirable balloon properties such as by minimizing drug loss and/or preventing hydration of a balloon coating (when a drug coated balloon is used). In cases where L₆ is equal to greater than L₄, the wall **1403** of the insertion tool can have a greater thickness, such as greater than about 0.025 mm, or greater than about 0.05 mm, such as in the range of about 0.05 mm to about 0.20 mm.

Figure 16d is a cross section of an insertion tool that has an inner dimeter of length L₇ that is greater than the maximum crossing profile L₄ of the folded balloon. In these embodiments, the folded balloon will fit in the inner diameter of the insertion tool and there will be some open space **1405** between the inner wall of the insertion tool and the outer surface of folded balloon. While this arrangement facilitates movement of the balloon portion of the balloon catheter within the insertion tool, preferably, the open space is limited to minimize backflow of blood. In some preferred embodiments, the maximum crossing profile of the folded balloon is about 80% or greater of the inner diameter of the insertion tool, about 82% or greater, about 84% or greater, about 86% or greater, about 88% or greater, about 90% or greater, about 92% or greater, about 94% or greater, about 96% or greater, or about 98% or greater of the inner diameter of the insertion tool, such as in the range of about 80% to about 99%, about 82% to about 99%, about 84% to about 99%, about 86% to about 99%, about 88% to about 99%, about 90% to about 99%, about 92% to about 99%, about 94% to about 99%, about 96% to about 99%, or about 98% to about 99% of the inner diameter of the insertion tool.

In a method of the disclosure, a balloon catheter can be inserted into a patient's body in a method that includes the following steps. First, (i) a balloon catheter comprising a catheter shaft and balloon portion having a length and (ii) an insertion tool for the entry of the balloon catheter into a patient's body, are provided. The insertion tool is one of the disclosure that has a proximal and distal ends along a lengthwise axis; a tubular portion extending proximally from the distal end having a length that is least the length of the balloon portion length, the tubular portion comprising: a wall, an inner diameter that can accommodate a balloon portion of a balloon catheter, a separation margin in the wall of a first lengthwise half of the tubular portion and which represents a structural weakening of, or a split in the wall in the first half; and a tab at the proximal end wherein either: (1) the tab extends from a second lengthwise half of the tubular portion, the tab having a portion at an angle skew to the lengthwise axis, or (2) the tab is in the form of a solid article comprising a concave surface that is fastened to an outer surface of the second lengthwise half of the tubular portion.

Next, the distal end of the insertion tool with balloon therein is inserted into in a hemostatic valve. After that, the tubular portion of the insertion tool and balloon catheter therein are advanced through the hemostatic valve and into the patient's body.

A portion of the proximal end of the insertion tool may remain proximal to the hemostatic valve during the insertion. With the insertion tool partially advanced through the hemostatic valve, the inflatable part of the balloon catheter can then be completely advanced through the insertion tool into the body.

Next, the tubular portion of the insertion tool is withdrawn from the hemostatic valve so as to position at least a proximal portion of the tubular portion around the catheter shaft which is proximal to the balloon portion. Next, the insertion tool is moved in relation to the balloon catheter to cause the separation margin to separate so the insertion tool can be moved away from the catheter shaft.

Alternatively, the insertion tool is placed proximal to inflatable portion of the balloon catheter, with the inflatable portion remaining protected by a separate protection sheath. The sheath can then be removed and the insertion tool then advanced over a folded inflatable part and then inserted through hemostatic valve with the balloon inside.

For insertion tool removal, force can be applied, for example, in an outward or proximal direction, to the tab of the insertion tool which causes the separation margin to separate so the insertion tool can be moved away from the catheter shaft. Alternatively the tubular portion of the insertion tool can be withdrawn all the way over the catheter shaft to the proximal hub, which can function as a wedge and cause the separation margin to separate, which in turn allows the insertion tool to be moved away from the catheter shaft. Alternatively, the user can move the insertion tool to the proximal portion of the balloon catheter which has an OD that is larger than the ID of the tubular part of the insertion tool. This portion of the catheter can then function as a wedge and can cause the insertion tool to split open along the separation margin.

Treatment of a plaque region is described in greater detail. The balloon portion of the balloon catheter can be inserted into the insertion tool and advanced through the hemostatic valve in an unexpanded state. A flared opening of the insertion tool may prevent loss of any coating on the balloon portion. After the guidewire is moved to a location for plaque treatment, the balloon portion is moved through the hemostatic valve, and the catheter with balloon portion is then fed over the guidewire until the balloon reaches the site for plaque treatment. The balloon can then be inflated at the plaque site thereby providing treatment. The manifold can also control the fluid introduction within shaft for expansion of the balloon.

The balloon is typically inflated using a fluid, which is injected through an inflation port. The mechanics of fluid transfer and introduction within balloons vary according to the specific design of the catheter, and are well known in the art.

In some embodiments, bioactive agent is associated with the surface of the balloon portion of the balloon catheter. The bioactive agent can be releasably associated with the balloon portion, or non-releasably associated with the balloon portion in a manner that it presents bioactive agent to body tissue. In some embodiments the balloon portion comprises a coating, such as hydrophilic or hydrogel coatings described herein, that can modulate the release of bioactive agent. For example, the bioactive agent can be present within and releasable from the polymeric material coated on the surface of the balloon. A polymeric coating may also be applied over a drug or drug containing layer to serve as a top coat which modulates the release of the bioactive agent.

Exemplary bioactive agents include, but are not limited to, antibiotics, antiinflammatory agents, anti-proliferative agents, immunomodulatory agents, anti-mitotics and anesthetics.

Examples of bioactive agents that could be released or presented from the balloon portion include sirolimus (rapamycin), analogs of rapamycin ("rapalogs"), tacrolimus, everolimus, zotarolimus, temsirolimus, pimecrolimus, ridaforolimus, paclitaxel, taxane, dexamethasone, betamethasone, paclitaxel, vinblastine, vincristine, vinorelbine, poside, teniposide, dactinomycin (actinomycin D), daunorubicin, doxorubicin, idarubicin, anthracyclines, mitoxantrone, bleomycins, plicamycin (mithramycin), mitomycin, mechlorethamine, cyclophosphamide and its analogs, melphalan, chlorambucil, ethylenimines and methylmelamines, alkyl sulfonates-busulfan, nirtosoureas, carmustine (BCNU) and analogs, streptozocin, trazenes-dacarbazinine, methotrexate, fluorouracil, floxuridine, cytarabine, mercaptopurine, thioguanine, pentostatin, 2-chlorodeoxyadenosine, cisplatin, carboplatin, procarbazine, hydroxyurea, mitotane, aminoglutethimide, estrogen, heparin, synthetic heparin salts, tissue plasminogen activator, streptokinase, urokinase, aspirin, dipyridamole, ticlopidine, clopidogrel, abciximab, breveldin, cortisol, cortisone, fludrocortisone, prednisone, prednisolone, 6U-methylprednisolone, triamcinolone, aspirin, acetaminophen, indomethacin, sulindac, etodalac, tolmetin, diclofenac, ketorolac, ibuprofen and derivatives, mefenamic acid, meclofenamic acid, piroxicam, tenoxicam, phenylbutazone, oxyphenthatrazone, nabumetone, auranofin, aurothioglucose, gold sodium thiomalate, cyclosporine, tacrolimus (FK-506), azathioprine, mycophenolate mofetil, vascular endothelial growth factor (VEGF), fibroblast growth factor (FGF); angiotensin receptor blocker; nitric oxide donors; anti-sense oligionucleotides and combinations thereof; cell cycle inhibitors, mTOR inhibitors, and growth factor signal transduction kinase inhibitors.

Other exemplary embodiments of bioactive agents include, but are not limited to plaque-penetrating peptides such as described by She et al., J. Contr. Rel. 238:212-220 , (2016), therapeutic antibodies, siRNA or microRNA (miRNA) targeting atherosclerosis (e.g., Feinberg et al., Circ Res. 118:703-20 (2016)).

Other exemplary embodiments of bioactive agents include, but are not limited to, bioactive agents for treatment of hypertension (HTN), such as guanethidine.

In a particular embodiment, the bioactive agents are selected from the group consisting of paclitaxel, sirolimus (rapamycin) and mixtures thereof.

In some embodiments, bioactive agent is associated with the balloon portion, and the bioactive agent is within, or in the form of microparticulates, that are associated with the balloon portion. When the second tube is expanded, the microparticulates can be released or dissociated from the balloon surface. Exemplary coatings include those including hydrophilic polymers, and those including degradable polymers. Following release from the balloon portion, the microparticulates can become associated with tissue and release bioactive agent.

In one embodiment, the plaque treatment portion comprises a flexible hydrogel coating and microparticulates associated with the balloon coating. The microparticulates may be associated with the coating in a non-homogenous manner, for example, the microparticulates can be associated with the flexible hydrogel coating (a) near the surface of the flexible hydrogel, coating, (b) predominantly near the flexible hydrogel coating/surface, or (c) homogenously distributed in the flexible hydrogel coating. Upon visualization, microparticulates that are marginally embedded in a flexible hydrogel coating may appear to be stuck to the coating surface. Exemplary balloon coatings including microparticulates with bioactive agent (e.g., paclitaxel) are described in U.S. Patent Nos. 8,951,545 and 9,669,192.

In one embodiment the balloon comprises a flexible hydrogel coating and on top a coating comprising a bioactive agent and an excipient. The excipient can function as a release agent or as an agent enhancing the drug transfer to the tissue to be treated. The excipient can be a polycation.

Microparticulates can be particulate components that include bioactive agent, and which are releasable from the surface of balloon portion. The microparticulates can be any three-dimensional particle having a size (e.g., in the range of about 100 nm to about 10 µm) and shape (spherical, or substantially spherical, non-spherical shapes or irregular shape, such as rod-like, filament-like, sliver-like, or needle-like shapes) sufficient to be associated with the surface via coating materials, and then dissociated upon its expansion of the balloon.

Microparticluates can comprise biocompatible materials that incorporate and/or encapsulate bioactive agent. These biocompatible materials can be biodegradable polymers (PLA, PLGA, etc.), (semi) solid lipids, biosilica, etc.

Microparticulates that are formed solely of one or more bioactive agents can be associated with the surface of the balloon and released to target tissue *in vivo.* In other words, the microparticulates can be formed substantially or entirely of one or more bioactive agents, and an excipient substance that may otherwise control release of the bioactive agent from the microparticulates is not required. A microparticulate that is formed entirely or almost entirely (e.g., allowing for trace amounts of one or more other components) of a bioactive agent may be referred to herein as a "neat" microparticulate.

The bioactive agent can be in amorphous form, in crystalline form or any mixture thereof.

For example, the preparation of paclitaxel microparticles has been described in U.S. Patent No. 6,610,317. Commonly assigned U.S. Application Serial Number 14/280,054 (U.S. 2014/0343491; Slager) and U.S. Application Serial Number 14/303,309 (U.S. 2015/0017219; Slager et al.) describes the preparation of macrolide particulates having desirable shapes and sizes using various solvent(s) and/or processing techniques.

## Claims

1. An insertion tool (1000) configured to facilitate entry of a balloon portion of a balloon catheter into a patient's body through a hemostatic valve, the insertion tool comprising
proximal (1014) and distal (1016) ends along a lengthwise axis;
a tubular portion (1012) extending proximally from the distal end having a length in the range of 10 mm to 300 mm, the tubular portion comprising:
a wall, an inner diameter in the range of 0.25 mm to 5 mm, first and second separation margins (1150, 1152) in the wall of the tubular portion representing structural weakenings of, or splits in the wall of the tubular portion, wherein the first and second separation margins define first and second lengthwise halves (1141, 1143) of the tubular portion; and
a first tab (1020) that extends from the first lengthwise half of the tubular portion and a second tab (1030) that extends from the second lengthwise half of the tubular portion, wherein the tubular portion extends a predetermined distance from the distal end to a point (1041) along its length where the first and second tabs begin, wherein the point is a split in the tubular member, and proximal to the split one half of the tubular member forms the first tab, and the other half of the tubular member forms the second tab,
wherein the first and second tabs have curved configurations relative to the lengthwise axis, an extent of a curve in the range of 45° to 180°, and curved tab radii in the range of 10 mm to 30 mm; and
the length of the tubular portion is two or more times the length of one or both tabs.

2. The insertion tool of claim 1, wherein the first and second separation margins (1150, 1152) comprise a fissure in the wall of the tubular member, the fissure having a depth that is at least 10% of the thickness of the wall, wherein the fissure optionally has a V shape.

3. The insertion tool of claim 1 or 2 having one or more of the following dimensions: a length of the tubular portion is in the range of about 10 mm to about 100 mm, about 50 mm to about 150 mm, or about 150 mm to about 300 mm; a circumference of the tubular portion is in the range of about 1.1 mm to about 32 mm, about 2.5 mm to about 15 mm, or about 3 mm to 6 mm; an outer diameter of the tubular portion is in the range of about 0.35 mm to about 10 mm, about 1.5 mm to about 5 mm, or about 1.6 mm to about 3 mm; the inner diameter of the tubular portion is in the range of about 1 mm to about 4 mm, about 1.2 mm to about 3 mm, or about 1.25 mm to about 2.75 mm; the thickness of the wall of tubular portion is in the range of about 0.05 mm to about 2.5 mm, about 0.05 mm to about 0.5 mm, about 0.05 mm to about 0.25 mm, about 0.05 mm to about 0.20 mm, about 0.05 mm to about 0.15 mm, about 0.10 mm to about 0.25 mm, or about 0.15 mm to about 1. 5 mm; and/or fissure depth in the range of about 0.025 mm to about 1 mm, about 0.05 mm to about 0.25 mm, or about 0.05 mm to about 0.15 mm.

4. The insertion tool of any of claims 1-3 comprising a material selected from the group consisting of fluorinated ethylene propylene (FEP); high and low density polyethylene (HDPE and LDPE), polytetrafluoroethylene (PTFE; Teflon); PEBAX, polyurethane; polyamide (Nylon); polyimide; and polyester.

5. The insertion tool of any of claims 1-4 wherein the tubular member has an outer and inner surface, which each independently or both can be associated with a low friction material, wherein optionally the low friction material is a hydrophilic coating or a fluoropolymer sleeve.

6. A balloon catheter insertion system comprising the insertion tool of any one of claims 1-5 and a balloon catheter, and optionally a hemostatic valve, wherein a balloon portion of the balloon catheter is optionally loaded into portion of the tubular portion of the balloon catheter, optionally the balloon portion comprises a balloon in a folded configuration and having a folded balloon diameter, and the folded balloon diameter is greater than, equal to, or less than the inner diameter of the tubular member,
optionally the folded balloon diameter is greater than the inner diameter of the tubular member, wherein the tubular member has a wall thickness of less than 0.075 mm, less than 0.06 mm, or less than 0.05 mm, or in the range of about 0.025 mm to about 0.06 mm, or about 0.025 mm to about 0.05 mm, and wherein the tubular member deforms to accommodate the folded balloon when introduced into the tubular member,
optionally the folded balloon diameter is less than inner diameter of the tubular member, and in the range of about 80% to about 99%, about 86% to about 99%, about 90% to about 99%, about 94% to about 99%, or about 96% to about 99% of the inner diameter of the tubular member.

7. The system of claim 6, wherein the balloon catheter comprises:
(a) a therapeutic agent on the balloon surface, the therapeutic agent optionally comprising rapamycin, or
(b) a polymeric coating on the balloon surface, the polymeric coating optionally comprising a hydrogel, or both (a) and (b).

## Patentansprüche

1. Ein Einführinstrument (1000), das dazu ausgelegt ist, das Einführen eines Ballonabschnitts eines Ballonkatheters in den Körper eines Patienten durch ein hämostatisches Ventil zu erleichtern, wobei das Einführinstrument umfasst:
ein proximales (1014) und ein distales (1016) Ende entlang einer Längsachse;
einen röhrenförmigen Abschnitt (1012), der sich vom distalen Ende aus proximal erstreckt und eine Länge im Bereich von 10 mm bis 300 mm aufweist, wobei der röhrenförmige Abschnitt umfasst:
eine Wand, einen Innendurchmesser im Bereich von 0,25 mm bis 5 mm, einen ersten und einen zweiten Trennrand (1150, 1152) in der Wand des röhrenförmigen Abschnitts, die strukturelle Schwächungen oder Spalten in der Wand des röhrenförmigen Abschnitts darstellen, wobei der erste und der zweite Trennrand eine erste und eine zweite Längshälfte (1141, 1143) des röhrenförmigen Abschnitts definieren; und
eine erste Lasche (1020), die sich von der ersten Längshälfte des röhrenförmigen Abschnitts erstreckt, und eine zweite Lasche (1030), die sich von der zweiten Längshälfte des röhrenförmigen Abschnitts erstreckt,
wobei sich der röhrenförmige Abschnitt über eine vorbestimmte Strecke vom distalen Ende bis zu einem Punkt (1041) entlang seiner Länge, an dem die erste und die zweite Lasche beginnen, erstreckt, wobei dieser Punkt eine Spalte im röhrenförmigen Abschnitt darstellt und proximal zur Spalte eine Hälfte des röhrenförmigen Abschnitts die erste Lasche bildet und die andere Hälfte des röhrenförmigen Abschnitts die zweite Lasche bildet,
wobei die erste und die zweite Lasche im Verhältnis zur Längsachse gekrümmt sind, einen Krümmungswinkel im Bereich von 45° bis 180° und Krümmungsradien im Bereich von 10 mm bis 30 mm aufweisen; und
die Länge des röhrenförmigen Abschnitts das Zweifache oder ein Vielfaches der Länge einer oder beider Laschen beträgt.

2. Das Einführinstrument nach Anspruch 1, wobei der erste und der zweite Trennrand (1150, 1152) einen Spalt in der Wand des rohrförmigen Abschnitts umfassen, wobei der Spalt eine Tiefe aufweist, die mindestens 10 % der Wandstärke beträgt, und wobei der Spalt gegebenenfalls eine V-Form aufweist.

3. Das Einführinstrument nach Anspruch 1 oder 2, das eine oder mehrere der folgenden Abmessungen aufweist: Die Länge des rohrförmigen Abschnitts liegt im Bereich von etwa 10 mm bis etwa 100 mm, etwa 50 mm bis etwa 150 mm oder etwa 150 mm bis etwa 300 mm; ein Umfang des rohrförmigen Abschnitts liegt im Bereich von etwa 1,1 mm bis etwa 32 mm, etwa 2,5 mm bis etwa 15 mm oder etwa 3 mm bis 6 mm; ein Außendurchmesser des röhrenförmigen Abschnitts liegt im Bereich von etwa 0,35 mm bis etwa 10 mm, etwa 1,5 mm bis etwa 5 mm oder etwa 1,6 mm bis etwa 3 mm; der Innendurchmesser des rohrförmigen Abschnitts liegt im Bereich von etwa 1 mm bis etwa 4 mm, etwa 1,2 mm bis etwa 3 mm oder etwa 1,25 mm bis etwa 2,75 mm; die Wandstärke des röhrenförmigen Abschnitts liegt im Bereich von etwa 0,05 mm bis etwa 2,5 mm, etwa 0,05 mm bis etwa 0,5 mm, etwa 0,05 mm bis etwa 0,25 mm, etwa 0,05 mm bis etwa 0,20 mm, etwa 0,05 mm bis etwa 0,15 mm, etwa 0,10 mm bis etwa 0,25 mm oder etwa 0,15 mm bis etwa 1,5 mm; und/oder eine Spalttiefe liegt im Bereich von etwa 0,025 mm bis etwa 1 mm, etwa 0,05 mm bis etwa 0,25 mm oder etwa 0,05 mm bis etwa 0,15 mm.

4. Das Einführinstrument nach einem der Ansprüche 1 bis 3, umfassend ein Material, das aus der Gruppe ausgewählt ist, bestehend aus fluoriertem Ethylen-Propylen (FEP); Polyethylen hoher und niedriger Dichte (HDPE und LDPE), Polytetrafluorethylen (PTFE; Teflon); PEBAX, Polyurethan; Polyamid (Nylon); Polyimid; und Polyester.

5. Das Einführinstrument nach einem der Ansprüche 1 bis 4, wobei der rohrförmige Abschnitt eine Außen- und eine Innenfläche aufweist, die jeweils unabhängig voneinander oder beide mit einem reibungsarmen Material versehen sein können, wobei das reibungsarme Material optional eine hydrophile Beschichtung oder eine Fluorpolymerhülse ist.

6. Ein Einführsystem für einen Ballonkatheter, umfassend das Einführinstrument nach einem der Ansprüche 1 bis 5 und einen Ballonkatheter sowie gegebenenfalls ein hämostatisches Ventil, wobei ein Ballonabschnitt des Ballonkatheters gegebenenfalls in einen Abschnitt des röhrenförmigen Teils des Ballonkatheters eingelegt ist,
wobei der Ballonabschnitt gegebenenfalls einen Ballon in gefalteter Konfiguration mit einem gefalteten Ballondurchmesser umfasst und der gefaltete Ballondurchmesser größer als, gleich oder kleiner als der Innendurchmesser des röhrenförmigen Abschnitts ist,
optional ist der gefaltete Ballondurchmesser größer als der Innendurchmesser des röhrenförmigen Abschnitts, wobei der röhrenförmige Abschnitt eine Wandstärke von weniger als 0,075 mm, weniger als 0,06 mm oder weniger als 0,05 mm hat oder im Bereich von etwa 0,025 mm bis etwa 0,06 mm oder etwa 0,025 mm bis etwa 0,05 mm liegt, und wobei sich der röhrenförmige Abschnitt verformt, um den gefalteten Ballon aufzunehmen, wenn dieser in den röhrenförmige Abschnitt eingeführt wird,
optional ist der Durchmesser des gefalteten Ballons kleiner als der Innendurchmesser des röhrenförmigen Abschnitts und im Bereich von etwa 80 % bis etwa 99 %, etwa 86 % bis etwa 99 %, etwa 90 % bis etwa 99 %, etwa 94 % bis etwa 99 % oder etwa 96 % bis etwa 99 % des Innendurchmessers des röhrenförmigen Abschnitts liegt.

7. Das System nach Anspruch 6, wobei der Ballonkatheter umfasst:
(a) ein therapeutisches Mittel auf der Ballonoberfläche, wobei das therapeutische Mittel gegebenenfalls Rapamycin umfasst, oder
(b) eine Polymerbeschichtung auf der Ballonoberfläche, wobei die Polymerbeschichtung gegebenenfalls ein Hydrogel umfasst, oder sowohl (a) als auch (b).

## Revendications

1. Un outil d'insertion (1000) configuré pour faciliter l'entrée d'une portion ballonneuse d'un cathéter ballonnet dans le corps du patient via une valve hémostatique, l'outil d'insertion comprenant
extrémités proximale (1014) et distale (1016) le long d'un axe longitudinal ;
une portion tubulaire (1012) s'étendant proximalement depuis l'extrémité distale d'une longueur comprise entre 10 mm et 300 mm, la portion tubulaire comprenant :
une paroi, d'un diamètre intérieur de 0,25 mm à 5 mm, des marges de séparation première et seconde (1150, 1152) dans la paroi de la partie tubulaire représentant des affaiblissements structurels ou des fissures dans la paroi de la partie tubulaire, où les première et deuxième marges de séparation définissent les deux moitiés longitudinales (1141, 1143) de la partie tubulaire ; et
une première languette (1020) qui s'étend depuis la première moitié longitudinale de la partie tubulaire et une seconde languette (1030) qui s'étend depuis la seconde moitié longitudinale de la partie tubulaire,
où la partie tubulaire s'étend sur une distance prédéterminée de l'extrémité distale jusqu'à un point (1041) le long de sa longueur où commencent la première et la deuxième languette, où la pointe est une fensure dans l'élément tubulaire, et proximal à la moitié fendue du membre tubulaire forme la première languette, et l'autre moitié forme la seconde,
où les premier et deuxième onglets ont des configurations courbes par rapport à l'axe longitudinal, une étendue d'une courbe allant de 45° à 180°, et des rayons de languette courbes allant de 10 mm à 30 mm ; et
la longueur de la partie tubulaire est deux fois ou plus la longueur d'une ou des deux languets.

2. L'outil d'insertion de la revendication 1, où les première et deuxième marges de séparation (1150, 1152) constituent une fissure dans la paroi du membre tubulaire, la fissure ayant une profondeur d'au moins 10 % de l'épaisseur du mur, où la fissure a éventuellement une forme en V.

3. L'outil d'insertion de la revendication 1 ou 2 ayant une ou plusieurs des dimensions suivantes : une longueur de la partie tubulaire est de l'ordre d'environ 10 mm à environ 100 mm, d'environ 50 mm à environ 150 mm, ou d'environ 150 mm à environ 300 mm ; une circonférence de la partie tubulaire est comprise entre environ 1,1 mm et environ 32 mm, environ 2,5 mm à environ 15 mm, ou environ 3 mm à 6 mm ; un diamètre extérieur de la partie tubulaire est de l'ordre d'environ 0,35 mm à environ 10 mm, d'environ 1,5 mm à environ 5 mm, ou d'environ 1,6 mm à environ 3 mm ; Le diamètre intérieur de la partie tubulaire est de l'ordre d'environ 1 mm à environ 4 mm, d'environ 1,2 mm à environ 3 mm, ou d'environ 1,25 mm à environ 2,75 mm ; L'épaisseur de la paroi de la partie tubulaire est comprise entre environ 0,05 mm et 2,5 mm, environ 0,05 mm à environ 0,5 mm, environ 0,05 mm à environ 0,25 mm, environ 0,05 mm à environ 0,20 mm, environ 0,05 mm à environ 0,15 mm, environ 0,10 mm à environ 0,25 mm, ou environ 0,15 mm à environ 1. 5 mm ; et/ou une profondeur de fissure dans la plage d'environ 0,025 mm à environ 1 mm, d'environ 0,05 mm à environ 0,25 mm, ou d'environ 0,05 mm à environ 0,15 mm.

4. L'outil d'insertion de l'une des revendications 1 à 3 comprenant un matériau sélectionné dans le groupe constitué d'éthylène propylène fluoré (FEP) ; polyéthylène haute et basse densité (HDPE et LDPE), polytétrafluoroéthylène (PTFE ; Téflon) ; PEBAX, polyuréthane ; polyamide (Nylon) ; polyimide ; et en polyester.

5. L'outil d'insertion de l'une des revendications 1 à 4 où le membre tubulaire possède une surface extérieure et une surface intérieure, chacune ou les deux pouvant être associées à un matériau à faible friction, où le matériau à faible friction est éventuellement un revêtement hydrophile ou un manchon fluoropolymère.

6. Un système d'insertion de cathéter ballonnet comprenant l'outil d'insertion de l'une des revendications 1 à 5 et un cathéter ballonnet, et éventuellement une valve hémostatique, dans laquelle une partie ballonneuse du cathéter ballonnet est éventuellement chargée dans une partie de la partie tubulaire du cathéter ballonnet,
Optionnellement, la partie ballon comprend un ballon en configuration pliée et ayant un diamètre de ballon plié, et le diamètre du ballon plié est supérieur, égal ou inférieur au diamètre intérieur de l'élément tubulaire,
Optionnellement, le diamètre du ballon plié est supérieur au diamètre intérieur de l'élément tubulaire, où l'élément tubulaire a une épaisseur de paroi inférieure à 0,075 mm, inférieure à 0,06 mm, ou inférieure à 0,05 mm, ou dans la plage d'environ 0,025 mm à environ 0,06 mm, ou d'environ 0,025 mm à environ 0,05 mm, et où l'élément tubulaire se déforme pour accueillir le ballon plié lorsqu'il est introduit dans l'élément tubulaire,
Optionnellement, le diamètre du ballon plié est inférieur au diamètre intérieur du membre tubulaire, et se situe dans la fourchette d'environ 80 % à environ 99 %, environ 86 % à environ 99 %, 94 % à 99 %, ou environ 96 % à 99 % du diamètre intérieur du membre tubulaire.

7. Le système de la revendication 6, dans lequel le cathéter ballonnet comprend :
(a) un agent thérapeutique à la surface du ballonnet, l'agent thérapeutique comprenant éventuellement la rapamycine, ou
(b) un revêtement polymérique à la surface du ballon, le revêtement polymérique comprenant éventuellement un hydrogel, ou les deux (a) et (b).
